# EUROPEAN PATENT SPECIFICATION

(11) **EP 2 956 770 B1**
(45) Date of publication and mention of the grant of the patent: **29.05.2019**
(21) Application number: 14714353.1
(22) Date of filing: 18.02.2014
(51) Int. Cl.: G01N 33/18, G01N 35/10

(54) **MODULAR PROBE-HOLDER**
MODULARER PROBENHALTER
PORTE-SONDE MODULAIRE

(30) Priority: 18.02.2013 IT RM20130090
(43) Date of publication of application: 23.12.2015
(73) Proprietor: SEKO S.p.A., 02010 Santa Rufina, Cittaducale (RI) (IT)
(72) Inventor: LIVOTI, Stefano, I-02010 Cittaducale (RI) (IT); DAMIANI, Andrea, I-02010 Cittaducale (RI) (IT); CORNACCHIOLA, Sergio, I-02010 Cittaducale (RI) (IT)
(74) Representative: Scilletta, Andrea
(86) International application number: PCT/IB2014/059063
(87) International publication number: WO 2014/125457

(56) References cited:
- EP-A2- 1 939 614
- US-A- 4 627 893
- US-A- 6 123 820
- US-A1- 2001 048 896
- US-A1- 2005 014 134
- US-A1- 2010 075 425
- US-A1- 2011 309 841
- US-B2- 6 886 421
- Aqua Water Systems: "Dosing Systems", , 1 January 2008 (2008-01-01), XP55409334, Retrieved from the Internet: URL:- [retrieved on 2017-09-22]
- Prominent Dosiertechnik Gmbh: "ProMinent Equipment", ProMinent Equipment Catalogue, 1 January 2004 (2004-01-01), pages 1-5, XP55280672,

## Description

The present invention relates to a modular probe-holder configured to house sensing probes for sensing physico-chemical parameters of a fluid, optionally water, that allows in a simple, reliable, efficient and inexpensive way to monitor and/or control the quality of the fluid adapting to different applications, the probe-holder being scalable in size and upgradeable in functionalities so as to render the installation and maintenance interventions simple, efficient, quick and inexpensive.

In the following of the present description, reference will be mainly made to applications of the modular probe-holder according to the invention to water treatment. However, it must understood that the modular probe-holder according to the invention may be applied in any other technological context in which the physico-chemical parameters of any fluid, even different from water, must be monitored and/or controlled, still remaining within the scope of protection of the present invention as defined in the attached claims.

It is known that in water treatment it is essential to sense physico-chemical parameters of the same water, such as for instance the volumetric flow rate, the pH value or the content of possibly dissolved specific substances (e.g., chlorine). To this end, sensing probes are used which are arranged along a sensing path in which water that is to undergo sensing at least partially flows. In particular, such sensing probes are housed in a dedicated compartment of a monolithic probe-holder, usually of plastic material, provided with an inlet and an outlet, that is part of such sensing path. An example of such probe-holders is the Pool Silver panel for controlling water in swimming pool, available from the Italian company Microdos s.r.l. and disclosed in the data sheet available in the web pages of the site of the same company at the address www.microdos.it; other examples of such probe-holders proposed in the prior art, even for different applications, are disclosed in documents US 4 550 011 A, DE 198 55 000 C1, US 2009/158819 A1 and US 2011/023586 A1. Also, US 4 627 893 A discloses a testing module for quantitative determination of concentrations of various ions in extremely small volumes of body fluids, such as blood samples; US 6,886,421 B2 discloses a modular diagnostic analyser for analysing biological fluids, such as blood samples.

Although the prior art probe-holders have been made for some decades, however they suffer from some drawbacks due to their poor adaptability to different application scenarios.

In fact, a specific application requires a peculiar arrangement of sensing probes, whereby it is necessary to make a corresponding specific probe-holder for each specific application, with consequent high manufacturing costs.

Moreover, in the case where an upgrade of an existing arrangement of sensing probes is required, it is necessary to make a new probe-holder, with consequent time delay for the upgrade (that could even entail a suspension of the water treatment) and high costs.

Furthermore, also maintenance of the sensing probes and probe-holder is not easy, again entailing long time and high costs.

It is an object of this invention, therefore, to allow in a simple, reliable, efficient and inexpensive way to render a probe-holder, configured to house sensing probes for monitoring and/or controlling the quality of water, and more generally of a fluid, scalable in size and upgradeable in functionalities, and hence adaptable to different applications, so as to permit simple, efficient, quick and inexpensive installation and maintenance interventions of both the probe-holder and the sensing probes which it is capable to house.

It is specific subject-matter of the present invention a modular probe-holder configured to house sensing probes for sensing physico-chemical parameters of a fluid, characterised in that it comprises two or more probe-holding modules, each one configured to house at least one sensing probe, which are laterally coupled in pairs so that each pair of laterally coupled probe-holding modules comprises a first probe-holding module, including a first duct connected to a first inlet and to a first outlet, and a second probe-holding module, including a second duct connected to a second inlet and to a second outlet, the first outlet being arranged on a first side wall of the first probe-holding module and the second inlet being arranged on a second side wall of the second probe-holding module, whereby the first side wall of the first probe-holding module is coupled to the second side wall of the second probe-holding module so that the first outlet communicates with the second inlet, each pair of laterally coupled probe-holding modules being configured to receive a flow of a fluid from the first inlet to the second outlet, the first and second probe-holding modules being coupled through fastening mechanical means inserted into at least one pair of facing seats, wherein a first seat is arranged on the first side wall of the first probe-holding module and a second seat is arranged on the second side wall of the second probe-holding module, at least one elastic seal being inserted into at least one seal groove, with which at least one from the first and second side walls is provided, said at least one seal groove being arranged for surrounding the first outlet and the second inlet, whereby said at least one elastic seal seals the coupling of the first side wall of the first probe-holding module to the second side wall of the second probe-holding module.

According to another aspect of the invention, said fastening mechanical means may comprise at least one tie inserted into said at least one pair of facing seats, the first seat communicating with a first threaded hole, that has a longitudinal axis and that is arranged on a front or rear wall of the first probe-holding module, into which a first grub screw is screwed, the second seat communicating with a second threaded hole, that has a longitudinal axis and that is arranged on a front or rear wall of the second probe-holding module, into which a second grub screw is screwed, said at least one tie comprising a first and a second holes having respective longitudinal axes and configured to receive a tip of the first and second grub screws, respectively, the tip of each one of the first and second grub screws having a conical lateral surface, optionally with apex angle equal to 90°, the first and second holes of said at least one tie having a support inclined surface corresponding to the conical lateral surface of the tip of each one of the first and second grub screws, a distance DA between the longitudinal axes of the first and second threaded holes being larger than the distance DB between the longitudinal axes of the first and second holes of said at least one tie, optionally by an amount ranging from 2% to 3%, more optionally by an amount equal to 2,5%, whereby when the tips of the first and second grub screws are received in the first and second holes of said at least one tie the first and second probe-holding modules are coupled to each other.

According to a further aspect of the invention, said fastening mechanical means may comprise at least one threaded stud screwed into said at least one pair of facing seats, whereby the first seat and the second seat are threaded.

According to an additional aspect of the invention, said at least one elastic seal may have circular cross-section having diameter w and said at least one seal groove has rectangular cross-section, having width w equal to the diameter w and depth p, wherein p ranges from 65% to 75% of w, p being optionally equal to 70% of w, said at least one elastic seal being optionally made of fluoroelastomer.

According to another aspect of the invention, the first duct and the second duct may be vertical, the first inlet, the first outlet, the second inlet and the second outlet being arranged so that the first duct and the second duct are configured to receive said flow of the fluid from the bottom upwards.

According to a further aspect of the invention, the first outlet may be superiorly arranged on the first side wall of the first probe-holding module and the second inlet may comprise a groove on the second right side wall of the second probe-holding module that communicates with the second duct through a lower transverse duct.

According to an additional aspect of the invention, each one of said two or more probe-holding modules may be a type of probe-holding module selected from a group comprising two or more types of probe-holding modules which are different and capable to be coupled to each other, optionally made of a transparent plastic material, more optionally made of poly(methyl methacrylate).

It is further specific subject-matter of the present invention a probe-holding module for use in the modular probe-holder as previously described, configured to house at least one sensing probe, that includes a duct connected to an inlet and to an outlet, the probe-holding module being configured to receive a flow of a fluid from the inlet to the outlet and having a first side wall and a second side wall, at least one from the inlet and the outlet being on one between the first and second side walls, the probe-holding module being characterised in that it is provided, on at least one between the first and second side walls, with at least one seat configured to receive fastening mechanical means.

According to another aspect of the invention, said at least one seat may be configured to partially receive at least one tie, said at least one seat communicating with at least one threaded hole arranged on a front or rear wall of the probe-holding module and configured to receive at least one grub screw.

According to a further aspect of the invention, said at least one seat may be screwed and it may be configured to receive at least one threaded stud.

According to an additional aspect of the invention, the probe-holding module may be further provided, on at least one between the first and second side walls on which the inlet or the outlet is present, with at least one seal groove configured to receive at least one elastic seal and arranged for surrounding the inlet or the outlet present on the side wall provided with said at least one seal groove, said at least one seal groove optionally having rectangular cross-section.

According to another aspect of the invention, the probe-holding module may be configured to house a flow meter, optionally comprising a rear hollow seat configured to house a proximity sensor, more optionally a Reed switch or a Hall effect sensor, the duct of the probe-holding module being a vertical duct configured to house a float interacting with a proximity sensor when the latter is housed in the rear hollow seat, the outlet of the probe-holding module being arranged on one between the first and second side walls, the inlet of the probe-holding module being a lower mouth and the outlet of the probe-holding module being superiorly arranged on one between the first and second side walls, the outlet of the probe-holding module being connected to the vertical duct through an upper transverse duct, the probe-holding module having an upper side hollow seat wherein a regulating tap threaded valve having a conical tip, the generatrices of which are inclined by an angle optionally ranging from 8° to 10° with respect to a longitudinal axis of the valve, the upper transverse duct being conical with generatrices inclined by an angle equal to the inclination angle of the generatrices of the conical tip of the valve, the valve optionally having a thread with pitch not larger than 1 mm.

According to a further aspect of the invention, the duct of the probe-holding module may be a vertical duct, the probe-holding module being configured to house an amperometric sensor in a lower hollow seat superiorly communicating with the vertical duct, the inlet of the probe-holding module comprising a groove on an inlet side wall selected between the first and second side walls, the groove communicating with the vertical duct through a lower transverse duct, the outlet of the probe-holding module being superiorly arranged on an outlet side wall selected between the first and second side walls differing from the inlet side wall, the probe-holding module being further provided with an upper transverse, optionally tubular, duct that puts in communication an upper portion of the groove with an upper portion of the vertical duct.

According to an additional aspect of the invention, the lower transverse duct may be connected between the groove and a lower mouth communicating externally, the lower hollow seat being configured to house a plurality of balls, the lower hollow seat communicating with the lower mouth through a plurality of inner tubular ducts inclined with respect to a transverse plane crossing the first and second side walls, an inclination angle α of each inner tubular duct with respect to said transverse plane optionally ranging from 20° to 30°, more optionally being equal to 25°, the lower hollow seat superiorly communicating with the vertical duct through one or more planar ducts oriented parallel to a longitudinal axis of the vertical duct.

According to another aspect of the invention, the probe-holding module may be made of transparent plastic material, optionally poly(methyl methacrylate), the module being optionally provided on a rear wall with at least one pair of holes, each hole being configured to receive a screw for fastening one or more rear brackets.

Optionally, the modular probe-holder is composed of two or more probe-holding modules coupled to each other the type of which is selected from a group comprising five types of different probe-holding modules capable to be coupled to each other, made of a transparent plastic material, optionally poly(methyl methacrylate) (also known with the acronym PMMA). The probe-holding modules of the modular probe-holder are configured to house sensing probes for sensing physico-chemical parameters of a fluid, optionally water, more optionally applicable in water treatment. The number of types of different probe-holding modules capable to be coupled to each other may be also different from five; by way of example, the probe-holding modules may be all of a sole type (in this case, the sole type of module may house some sensing probes configured to be received by such sole probe-holding module), or the types of probe-holding modules may be two or more than two.

The modularity of the probe-holder according to the invention is based on single probe-holding modules which mechanically couple to each other through threaded members.

Also the fastening of the modular probe-holder favours its modularity, through suitable, optionally plastic, supports fastened to the end probe-holding modules of the modular probe-holder.

The advantages offered by the modular probe-holder according to the invention are evident.

First of all, the flow of water (or other fluid) proceeds along a constrained path within the modular probe-holder through suitable passages obtained between the probe-holding modules, so that the sensing probes receive the water flow from the bottom upwards. The modular probe-holder allows to keep a constant velocity of the flow along the entire path for rendering the same adapted to the different sensing probes housed in the probe-holding modules.

Some embodiments of the modular probe-holder according to the invention comprise a particular type of probe-holding module that is configured to house a horizontal amperometric sensor and also to carry out a degassing of the fluid through the ducts which exploit the Venturi effect.

The probe-holding modules coupled to each other ensures performance of the modular probe-holder up to a pressure equal to 7 bar (where 1 bar = 10⁵ Pascal) and up to a temperature of 70°C.

The probe-holding modules practically allows unlimited combinations of the single probe-holding modules allowing a modular probe-holder, advantageously applicable to water treatment, to be made that is capable to house different probes for sensing physico-chemical parameters of a fluid, the modular probe-holder being thus scalable in size and upgradeable in functionalities, also rendering the installation and maintenance interventions efficient, with consequent reduction of time and costs.

The present invention will be now described, by way of illustration and not by way of limitation, according to its preferred embodiments, by particularly referring to the Figures of the annexed drawings, in which:
Figure 1 shows a right side view of a first type of probe-holding module (Fig. 1a), a front view of a second type of probe-holding module (Fig. 1b), a front view of a third type of probe-holding module (Fig. 1c), a front view of a fourth type of probe-holding module (Fig. 1d), and a front view of a fifth type of probe-holding module (Fig. 1e) of the preferred embodiment of the modular probe-holder according to the invention;
Figure 2 shows a front perspective view of the first type of probe-holding module (Fig. 2a) and a rear perspective view of the fifth type of probe-holding module (Fig. 2b) of Figure 1;
Figure 3 shows a front view (Fig. 3a) and a cross-section view along the plane AA of Figure 3a (Fig. 3b) of a first assembly of four of the probe-holding modules of Figure 1;
Figure 4 shows a first cross-section view (Fig. 4a) and a second cross-section view (Fig. 4b) of a particular of a coupling zone between two probe-holding modules of Figure 1, wherein each view represents specific features of the coupling;
Figure 5 shows a left side view of the fifth type of probe-holding module (Fig. 5a) of Figure 1 and a cross-section view (Fig. 5b) of a particular of a coupling zone between two probe-holding modules of Figure 1;
Figure 6 shows a front view (Fig. 6a) and a cross-section view along the plane BB of Figure 6a (Fig. 6b) of an assembly of four probe-holding modules of a further embodiment of the modular probe-holder according to the invention;
Figure 7 shows a cross-section view (Fig. 7a) of the first assembly of Figure 3 wherein each probe-holding module houses a respective sensing probe, an enlargement of the cross-section view (Fig. 7b) and a perspective view of a component coupled to the first assembly (Fig. 7c);
Figure 8 shows a rear perspective view of a second assembly of two of the probe-holding modules of Figure 1 wherein each probe-holding module houses a respective sensing probe;

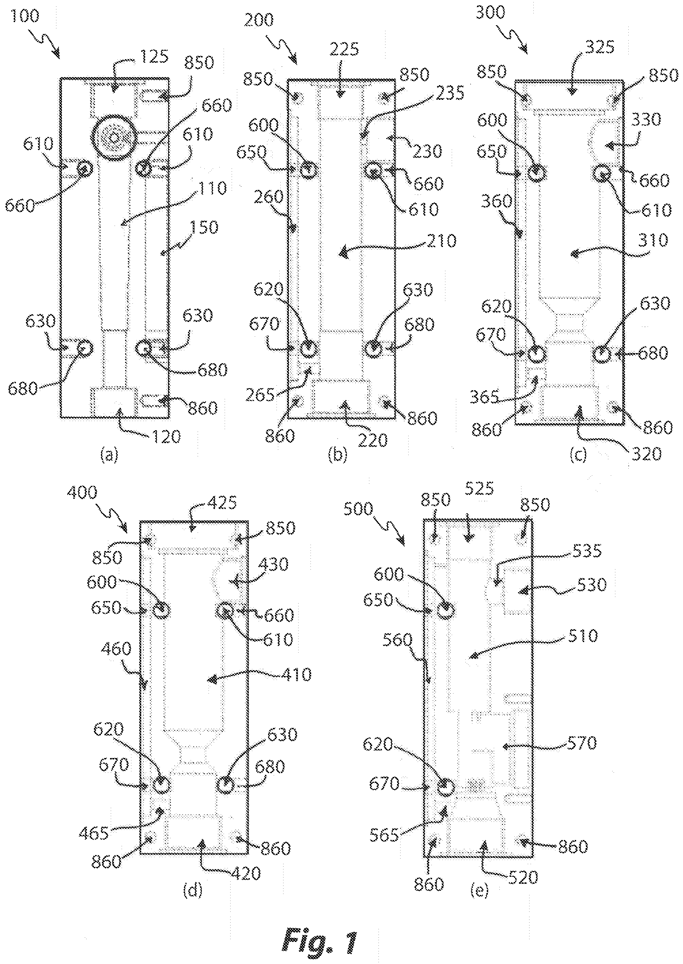

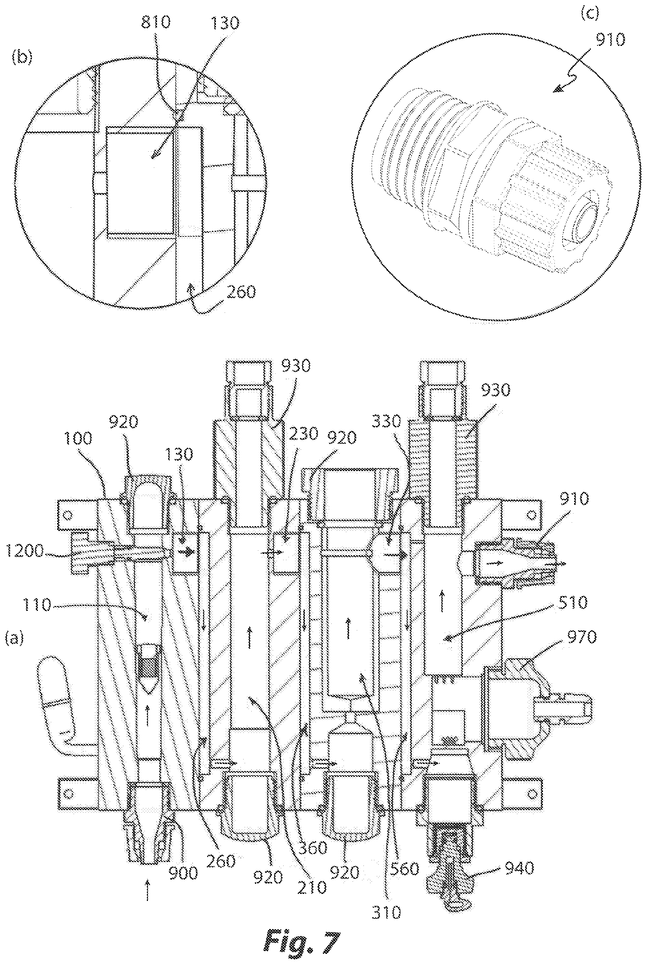

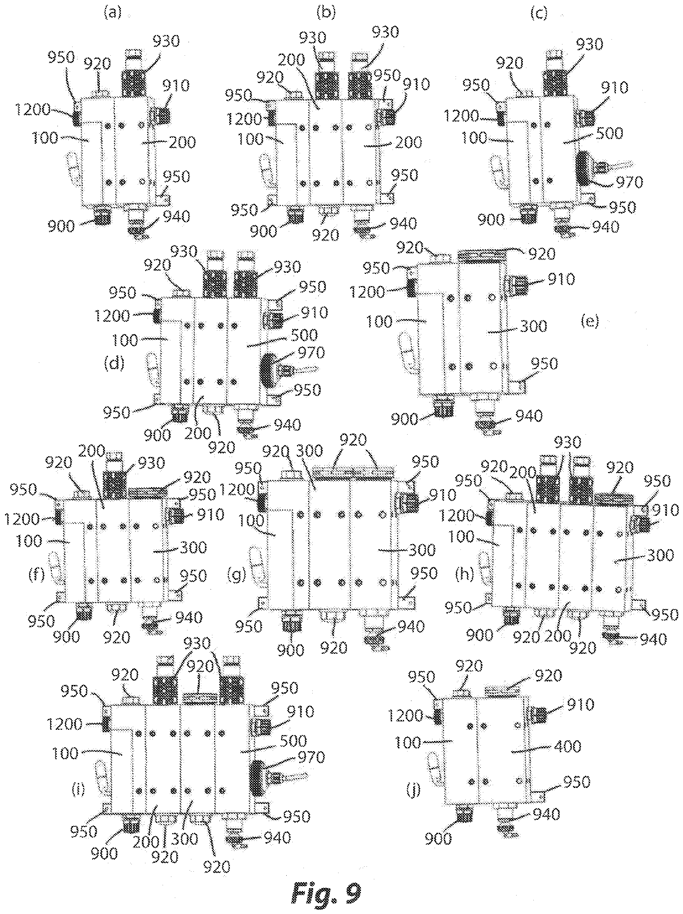

Figure 9 shows ten front perspective views of a third assembly of two of the probe-holding modules of Figure 1 (Fig. 9a), of a fourth assembly of three of the probe-holding modules of Figure 1 (Fig. 9b), of a fifth assembly of two of the probe-holding modules of Figure 1 (Fig. 9c), of a sixth assembly of three of the probe-holding modules of Figure 1 (Fig. 9d), of a seventh assembly of two of the probe-holding modules of Figure 1 (Fig. 9e), of an eighth assembly of three of the probe-holding modules of Figure 1 (Fig. 9f), of a ninth assembly of three of the probe-holding modules of Figure 1 (Fig. 9g), of a tenth assembly of four of the probe-holding modules of Figure 1 (Fig. 9h), of the first assembly of Figure 7 (Fig. 9i), and of an eleventh assembly of two of the probe-holding modules of Figure 1 (Fig. 9j), wherein each probe-holding module houses a respective sensing probe;
Figure 10 shows a cross-section view of the first type of probe-holding module of Figure 1;
Figure 11 shows a block diagram schematically representing a control executed by an electronic control unit connected to the sensing probes housed in the first type of probe-holding module and in the fifth type of probe-holding module of Figure 1;
Figure 12 shows an enlargement of a portion of a cross-section of the third assembly of Figure 9a (Fig. 12a), an enlargement of a particular (Fig. 12b) of the portion of Figure 12a, and a perspective view of a component (Fig. 12c) coupled to the third assembly of Figure 12a; and
Figure 13 shows an enlargement of a portion of the cross-section of the first assembly of Figure 7 (Fig. 13a), a cross-section view along the plane CC of Figure 13a (Fig. 13b) and two enlargements of the same particular of Figure 13b (Fig. 13c and Fig. 13d), wherein each enlargement represents specific features of the particular.

In the Figures identical reference numerals will be used for alike elements.

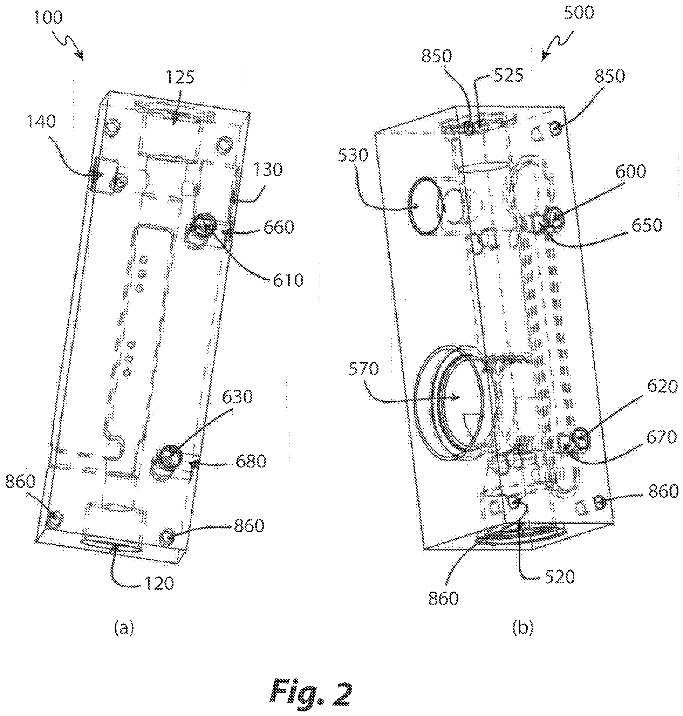

With reference to Figures 1 and 2, it may be observed that the preferred embodiment of the modular probe-holder according to the invention may be composed of two or more probe-holding modules coupled to each other the type of which is selected from a group comprising five types of different probe-holding modules, each one made in one piece of plexiglass that can be inscribed in a substantially parallelepiped-shaped polyhedron, and capable to be coupled to each other.

A first type of probe-holding module 100, shown in Figures 1a and 2a, is configured to house an adjustable flow meter, optionally capable to measure a volumetric flow rate ranging from 10 to 100 litres/hour. The first probe-holding module 100 comprises a longitudinal central duct (i.e., a vertical central duct) 110 that communicates externally through a lower threaded mouth 120, through an upper hollow threaded seat 125 and through an upper right side threaded outlet 130 ("right side" with respect to the front view of the first probe-holding module 100) connected to the longitudinal central duct 110 through an upper right transverse duct 135. Moreover, the first probe-holding module 100 is provided with an upper left side hollow seat 140 and a rear hollow seat 150.

A second type of probe-holding module 200, shown in Figure 1b, is configured to house a sensing probe and comprises a longitudinal central duct 210 having a first diameter, optionally equal to 12 mm, that communicates externally through a lower threaded mouth 220, through an upper hollow threaded seat 225 and through an upper right side threaded outlet 230 ("right side" with respect to the front view of the second probe-holding module 200) connected to the longitudinal central duct 210 through an upper right transverse duct 235. Moreover, the second probe-holding module 200 is provided with a groove 260 on the left side wall that communicates with the longitudinal central duct 210 through a lower left transverse duct 265.

A third type of probe-holding module 300, shown in Figure 1c, is configured to house a sensing probe and comprises a longitudinal central duct 310 having a second diameter larger than the first diameter, optionally equal to 24 mm, that communicates externally through a lower threaded mouth 320, through an upper hollow threaded seat 325 and through an upper right side threaded outlet 330 ("right side" with respect to the front view of the third probe-holding module 300). Moreover, the third probe-holding module 300 is provided with a groove 360 on the left side wall that communicates with the longitudinal central duct 310 through a lower left transverse duct 365.

A fourth type of probe-holding module 400, shown in Figure 1d, is configured to house a sensing probe and comprises a longitudinal central duct 410 having a third diameter larger than the second diameter, optionally equal to 35 mm, that communicates externally through a lower threaded mouth 420, through an upper hollow threaded seat 425 and through an upper right side threaded outlet 430 ("right side" with respect to the front view of the fourth probe-holding module 400). Moreover, the fourth probe-holding module 400 is provided with a groove 460 on the left side wall that communicates with the longitudinal central duct 410 through a lower left transverse duct 465.

A fifth type of probe-holding module 500, shown in Figures 1e and 2b, is configured to house a horizontal amperometric sensor in a lower right side hollow seat 570 communicating inferiorly with a lower threaded mouth 520, in turn communicating externally, and superiorly with a longitudinal duct 510, in turn communicating externally through an upper hollow threaded seat 525 and through an upper right side threaded outlet 530 ("right side" with respect to the front view of the fifth probe-holding module 500). Moreover, the fifth probe-holding module 500 is provided with a groove 560 on the left side wall that communicates with the lower right side hollow seat 570 through a lower left transverse duct 565.

The fastening of the probe-holding modules is guaranteed by ties constrained in the probe-holding modules operated by grub screws, allowing the coupling between probe-holding modules without constraints of consecutiveness apart from the first type of probe-holding module 100 that, in the preferred embodiment of the modular probe-holder, must be always the first module of the series of assembled modules which form the modular probe-holder and apart from the fifth type of probe-holding module 500 that, in the preferred embodiment of the modular probe-holder, must be always the last module of the series of assembled modules which form the modular probe-holder.

To this end, the second, the third and the fourth type of probe-holding modules 200, 300 and 400 are provided on the front and rear walls with a pair of upper left threaded holes 600 communicating with a pair of respective upper left side seats 650 communicating externally and having longitudinal axis orthogonal to the axis of the respective threaded hole 600, a pair of upper right threaded holes 610 communicating with a pair of respective upper right side seats 660 communicating externally and having longitudinal axis orthogonal to the axis of the respective threaded hole 610, a pair of lower left threaded holes 620 communicating with a pair of respective lower left side seats 670 communicating externally and having longitudinal axis orthogonal to the axis of the respective threaded hole 620, and a pair of lower right threaded holes 630 communicating with a pair of respective lower right side seats 680 communicating externally and having longitudinal axis orthogonal to the axis of the respective threaded hole 630; each one of the threaded holes is configured to receive a grub screw, while each one of the seats communicating with the threaded holes is configured to partially receive a tie.

The first type of probe-holding module 100 is provided on the front and rear walls with only the pair of upper right threaded holes 610 and the pair of lower right threaded holes 630, each one of which is also configured to receive a grub screw, communicating with respective upper and lower right side seats 660 and 680, each one of which is also configured to partially receive a tie.

The fifth type of probe-holding module 500 is provided on walls front and rear walls with only the pair of upper left threaded holes 600 and the pair of lower left threaded holes 620, each one of which is also configured to receive a grub screw, communicating with respective upper and lower left side seats 650 and 670, each one of which is also configured to partially receive a tie.

However, it should be understood that other embodiments of the modular probe-holder according to the invention may have probe-holding modules configured to house amperometric sensor (for instance a horizontal amperometric sensor housed in a front hollow seat or a vertical amperometric sensor housed in a corresponding seat) and which may be coupled to other modules in any position along the series of assembled modules forming the modular probe-holder (i.e. not necessarily placed as last module); in this case, such probe-holding modules configured to house amperometric sensors are provided with the same assembly of pairs of threaded holes 600, 610, 620 and 630 and of communicating seats 650, 660, 670 and 680 with which the second, the third and the fourth type of probe-holding modules 200, 300 and 400 are provided.

Moreover, other embodiments of the modular probe-holder according to the invention may have probe-holding modules configured to house flow meters which may be coupled to other modules in any position along the series of assembled modules forming the modular probe-holder (i.e. not necessarily placed as first module); such probe-holding modules configured to house flow meters are also provided with the same assembly of pairs of threaded holes 600, 610, 620 and 630 and of communicating seats 650, 660, 670 and 680 with which the second, the third and the fourth type of probe-holding modules 200, 300 and 400 are provided.

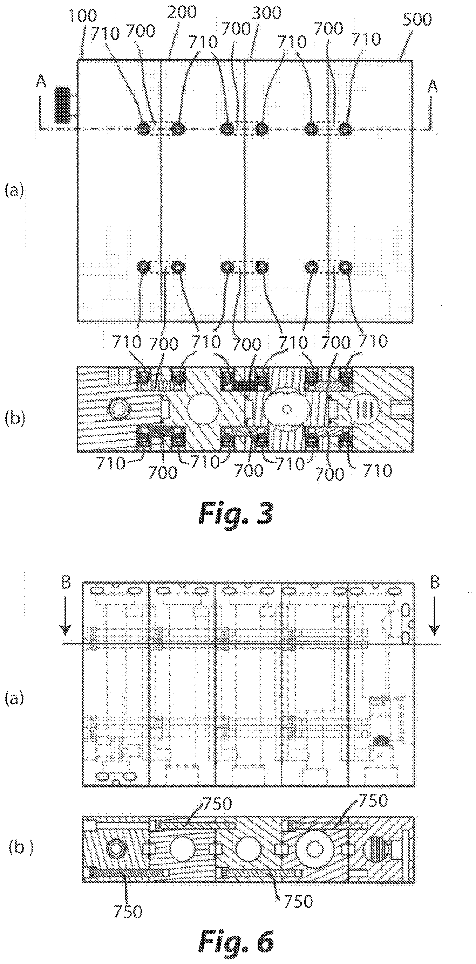

With reference to Figure 3, wherein by way of example and not by way of limitation a modular probe-holder constituted of an ordered series of a first type, a second type, a third type and a fifth type of probe-holding modules 100, 200, 300 and 500 is shown, it may be observed that the ties 700 are inserted into the pairs of facing side seats 650, 660, 670 and 680 of two adjacent probe-holding modules and afterwards the grub screws 710 are screwed into the threaded holes 600, 610, 620 and 630, causing the adjacent probe-holding modules to get closer. As stated, such fastening is present on both front and rear faces of the probe-holding modules.

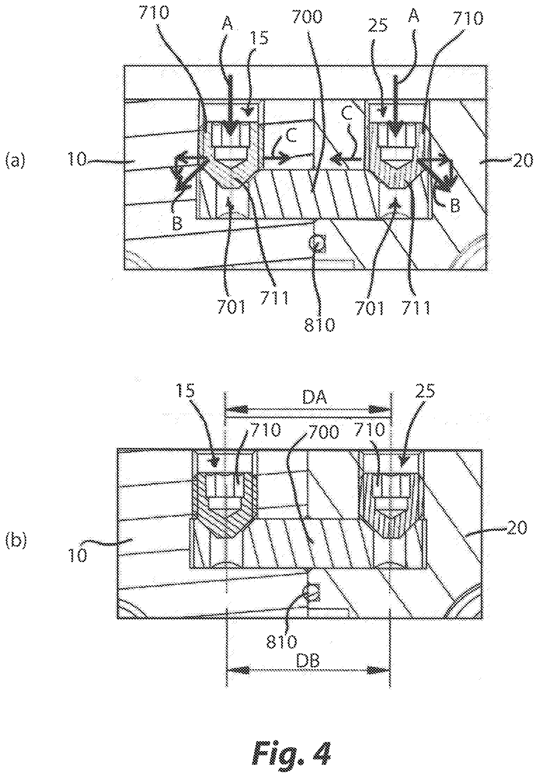

In order to better understand the operation of the grub screws 700 and ties 710 reference can be made to Figure 4, wherein an enlargement of a cross-section of a coupling zone of two adjacent probe-holding modules (generically indicated with the reference numerals 10 and 20) comprising a tie 700 inserted into a pair of corresponding facing side seats is shown. The tie 700 comprises two holes 701 each configured to receive the tip 711 of a grub screw 710 screwed into a respective threaded hole (the threaded holes are generically indicated with the reference numerals 15 and 25). In particular, the tip 711 of a grub screw has a conical lateral surface, optionally with apex angle equal to 90° (whereby the side surface has an inclination of 45° with respect to the longitudinal axis of the grub screw 710), and the hole 701 and tie 700 have a corresponding support inclined surface, optionally with inclination of 45° with respect to the longitudinal axis of the hole 701.

The area of contact between tip 711 of the grub screw 710 and hole 701 of the tie 700 only comprises the portion of the support inclined surface of the hole 701 of the tie 700 that is farthest from the adjacent probe-holding module (20 or 10). To this end, when the tie 700 is symmetrically arranged in the pair of corresponding facing side seats, there is an offset between the longitudinal axis of each one of the threaded holes 15 and 20 (coinciding with the longitudinal axis of the grub screw 710 inserted into such threaded hole) and the longitudinal axis of the hole 701 of the tie 700; optionally, when the two adjacent probe-holding modules 10 and 20 are coupled, the distance DA between the longitudinal axes of the two threaded holes 15 and 20 (communicating with the pair of side seats facing the two adjacent probe-holding modules 10 and 20) is larger than the distance DB between the longitudinal axes of the holes 701 of the tie 700, optionally by an amount ranging from 2% to 3%, more optionally by an amount equal to 2,5% (whereby DA=1,025*DB).

When a grub screw 710 is screwed into the respective threaded hole (15 or 25), it advances longitudinally (along the direction of the arrow A) exerting a force (along the direction of the arrow B) perpendicular to the support surface of the respective hole 701 of the tie 700. The preferred inclination angle of the side surface of the tip 711 of the grub screw, equal to 45°, maximises the area of contact between tip 711 of the grub screw 710 and hole 701 of the tie 700 dedicated to the transmission of forces. The horizontal component of such force produces a constraint reaction (along the direction of the arrow C) on the portion of the surface of the threaded hole (15 or 25) closest to the adjacent probe-holding module (20 or 10), causing the two adjacent probe-holding modules 10 and 20 to get closer to each other.

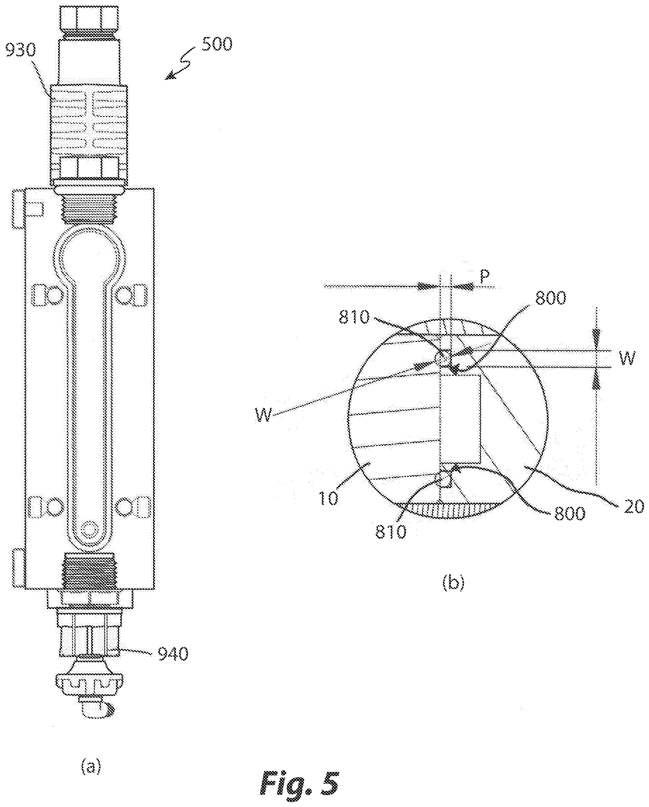

With reference to Figures 2b and 5a, wherein by way of example and not by way of limitation a probe-holding module 500 of the fifth type configured to house an amperometric sensor is shown, it may be observed that on the left side wall of the probe-holding modules is present a seal groove 800 arranged so as to surround the groove 560 of the probe-holding module 500 and, when the latter is coupled to an adjacent probe-holding module (100, 200, 300 or 400), also the upper left side outlet (130, 230, 330 or 430) on the left side wall of the adjacent probe-holding module (100, 200, 300 or 400). The seal groove 800 is configured to house an O-ring seal 810 of elastic material, optionally of fluoroelastomer known as FPM or FKM, with optionally circular cross-section, whereby the seal 810 is capable to ensure the sealing of the coupling between adjacent probe-holding modules since it surrounds the path of the fluid when passing from one to the other of the two adjacent probe-holding modules.

As shown in Figure 5b, wherein an enlargement of a cross-section of a coupling zone of two adjacent probe-holding modules (generically indicated with the reference numerals 10 and 20) comprising a groove 800 housing a seal 810 is shown, the geometry and size of the groove 800 and seal 810 are such that they guarantee the compression of the seal 810 when the two adjacent probe-holding modules 10 and 20 are coupled, so as to compensate planarity errors (i.e. irregularities of the opposing surfaces) of the facing walls of the two adjacent probe-holding modules 10 and 20. In this way, the seal 810 ensures the correct operation of the modular probe-holder with both fluid in static conditions and moving flow under several operation conditions, for instance at a first condition with fluid temperature of 25°C and fluid pressure of 10 bar and at a second condition with fluid temperature of 70°C and fluid pressure of 7 bar. Advantageously, the deformation of the seal 810 mainly concerns the direction orthogonal to the walls of contact of two adjacent probe-holding modules 10 and 20. Optionally, the groove 800 has rectangular cross-section, with depth p and width w equal to the diameter w of the seal 810 with circular cross-section, wherein p ranges from 65% to 75% of w (whereby 0.65*w ≤ p ≤ 0,7*w), p being more optionally equal to 70% of w (whereby p=0,7*w). In this case, even when the single probe-holding modules provided with the seal housed in the groove are disassembled, the seal stably remains in the groove even if the module is moved. In other words, the sealing effect is given by the deformation of the seal 810 during fastening of two adjacent probe-holding modules 10 and 20. The specific torque of fastening of the grub screws 710 and the absence of vibrations during the operation of the modular probe-holder do not render the use of self-locking devices necessary and ensure the compression imposed to the seal 810 for the entire lifetime of the modular probe-holder. Optionally, the hardness of the seal is not lower than 50 shore A (SHA), more optionally not lower than 60 SHA, in order to avoid that the seal is extruded between the walls of the two adjacent probe-holding modules 10 and 20.

In the preferred embodiment of the modular probe-holder, since the first type of probe-holding module 100 is the first module of the series of assembled modules forming the modular probe-holder, such first type of probe-holding module 100 is not provided on the left side wall with the seal groove 800.

Other embodiments of the modular probe-holder according to the invention may have the probe-holding modules having a seal groove on the right side wall, instead of the left side wall, still arranged so as to surround the path of the fluid when passing from one to the other of the two adjacent probe-holding modules. In the case where the fifth type of probe-holding module 500 is still always the last module of the series of assembled modules forming a modular probe-holder, such fifth type of probe-holding module 500 would not be provided on the right side wall with such seal groove.

Further embodiments of the modular probe-holder according to the invention may have the probe-holding modules which have a seal groove on both the left side wall and the right side wall, configured such that they do not overlap with the seal groove respectively on the right side wall and the left side wall of two other adjacent modules, both the seal grooves on the two side walls being arranged so as to surround the path of the fluid when passing from one to the other of the two adjacent probe-holding modules.

It should be understood that the specific arrangement of the seal groove configured to house an O-ring seal, illustrated with reference to Figures 2b and 5, is applicable to any hydraulic coupling of two elements through respective walls on which two apertures which can create a passage for a fluid flowing from one element to the other are present.

With reference to Figure 6, it may be observed that in a further embodiment of the modular probe-holder according to the invention, instead of using grub screws and ties, the probe-holding modules may be coupled to each other in pairs with threaded studs 750 screwed in suitable transverse through holes (operating as side seats) accessible on the side walls of the single probe-holding modules. Differently from the solution adopted in the preferred embodiment shown in Figures 3 and 4, the coupling through threaded studs 750 imposes that, in order to access a probe-holding module of a series of assembled modules forming the modular probe-holder, it is necessary to disassemble all the subsequent probe-holding modules.

Figure 7, wherein by way of example and not by way of limitation a modular probe-holder constituted by an ordered series of a first type, a second type, a third type and a fifth type of probe-holding module 100, 200, 300, and 500 (illustrated with reference to Figures 1 and 2) is shown, each one of which houses a respective sensing probe, shows the path of the water flow in the modular probe-holder according to the invention (schematically represented by the arrows in Figure 7). In particular, the entrance of the water flow in the modular probe-holder occurs through a tube-holder 900 coupled in the lower threaded mouth 120 of the first probe-holding module 100. The longitudinal ducts 110, 210, 310 and 510 of the probe-holding modules 100, 200, 300, and 500 guarantee that the fluid is moved from the bottom upwards inside each probe-holding module and that it has a constant velocity along the entire probe-holding module. The exit of the flow from each probe-holding module always occurs from the right wall of the same module, in particular from the upper right side outlets 130, 230, 330 and 530 of the probe-holding modules 100, 200, 300, and 500. The path of the fluid when passing from the previous to the next of two adjacent probe-holding modules occurs along the grooves on the left side walls 260, 360 and 560 of the next probe-holding module, as shown by the enlarged particular of Figure 7b related to the passage from the second probe-holding module 200 to the third probe-holding module 300. The flow exiting from the last probe-holding module of the series, that in Figure 7 is the fifth probe-holding module 500, is channelled by screwing a tube-holder 910 (shown enlarged in Figure 7c) into the upper right side outlet. The lower threaded mouths 220, 320 and 520 of the second, third and fifth probe-holding modules 200, 300, and 500, as well as the upper threaded mouths 125, 225, 325 and 525 of all the probe-holding modules 100, 200, 300, and 500 are closed by caps 920 and other devices 930 and 940 (e.g. electrodes and taps), whereby they do not provide the flow shown in Figure 7 with other exits.

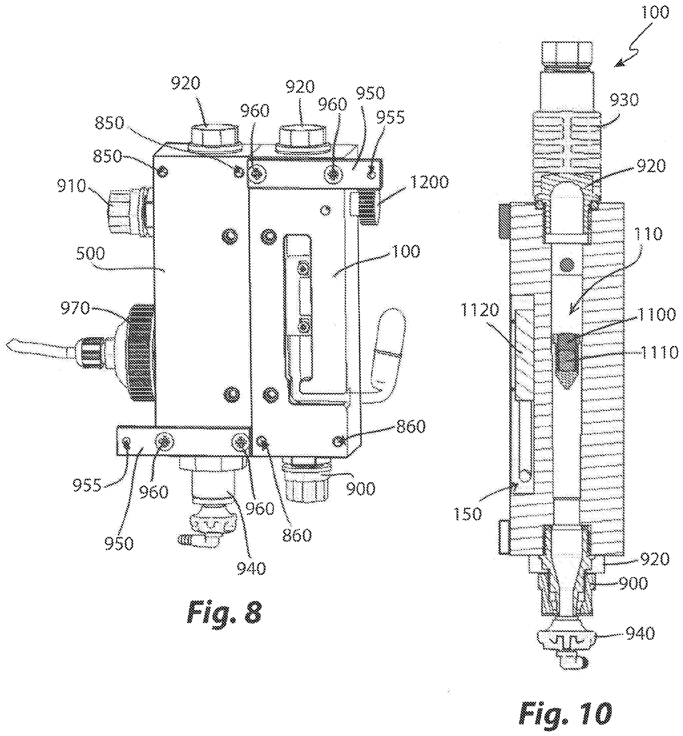

Making reference again to Figures 1 and 2, all the probe-holding modules are provided, on the rear wall, with a pair of upper rear threaded holes 850 and with a pair of lower rear threaded holes 860 configured to receive a screw for fastening rear brackets allowing the modular probe-holder to be fixed to a wall or support plate. Such rear brackets are fixed to the probe-holding modules at the ends of the series of assembled modules forming the modular probe-holder. By way of example, and not by way of limitation, Figure 8 shows a modular probe-holder constituted by a first type and a fifth type of probe-holding modules 100 and 500 to which two rear brackets 950 have been fixed through screws 960 inserted into the rear threaded holes 850 and 860; the rear brackets 950 are provided with holes 955 for the fastening through conventional means, such as Fischer plugs, to a wall or support plate.

The probe-holding modules allow to make modular probe-holders having any configuration. By way of example, Figure 9 shows:
- in Figure 9a, a modular probe-holder composed of a probe-holding module 100 of the first type, coupled to an inlet tube-holder 900 and housing a flow meter, and a probe-holding module 200 of the second type, coupled to an outlet tube-holder 910 and provided with a tap 940 for sampling water;
- in Figure 9b, a modular probe-holder composed of a probe-holding module 100 of the first type, coupled to an inlet tube-holder 900 and housing a flow meter, and two probe-holding modules 200 of the second type, the last of which is coupled to an outlet tube-holder 910 and is provided with a tap 940 for sampling water;
- in Figure 9c, a modular probe-holder composed of a probe-holding module 100 of the first type, coupled to an inlet tube-holder 900 and housing a flow meter, and a probe-holding module 500 of the fifth type, that houses a horizontal amperometric sensor 970 and is coupled to an outlet tube-holder 910 and is provided with a tap 940 for sampling water;
- in Figure 9d, a modular probe-holder composed of a probe-holding module 100 of the first type, coupled to an inlet tube-holder 900 and housing a flow meter, a probe-holding module 200 of the second type, and a probe-holding module 500 of the fifth type, that houses a horizontal amperometric sensor 970 and is coupled to an outlet tube-holder 910 and is provided with a tap 940 for sampling water;
- in Figure 9e, a modular probe-holder composed of a probe-holding module 100 of the first type, coupled to an inlet tube-holder 900 and housing a flow meter, and a probe-holding module 300 of the third type, coupled to an outlet tube-holder 910 and provided with a tap 940 for sampling water;
- in Figure 9f, a modular probe-holder composed of a probe-holding module 100 of the first type, coupled to an inlet tube-holder 900 and housing a flow meter, a probe-holding module 200 of the second type, and a probe-holding module 300 of the third type, coupled to an outlet tube-holder 910 and provided with a tap 940 for sampling water;
- in Figure 9g, a modular probe-holder composed of a probe-holding module 100 of the first type, coupled to an inlet tube-holder 900 and housing a flow meter, and two probe-holding modules 300 of the third type, the last of which is coupled to an outlet tube-holder 910 and is provided with a tap 940 for sampling water;
- in Figure 9h, a modular probe-holder composed of a probe-holding module 100 of the first type, coupled to an inlet tube-holder 900 and housing a flow meter, two probe-holding modules 200 of the second type, and a probe-holding module 300 of the third type, coupled to an outlet tube-holder 910 and provided with a tap 940 for sampling water;
- in Figure 9i, a modular probe-holder composed of a probe-holding module 100 of the first type, coupled to an inlet tube-holder 900 and housing a flow meter, a probe-holding module 200 of the second type, a probe-holding module 300 of the third type, and a probe-holding module 500 of the fifth type, that houses a horizontal amperometric sensor 970 and is coupled to an outlet tube-holder 910 and is provided with a tap 940 for sampling water; and
- in Figure 9j, a modular probe-holder composed of a probe-holding module 100 of the first type, coupled to an inlet tube-holder 900 and housing a flow meter, and a probe-holding module 400 of the fourth type, coupled to an outlet tube-holder 910 and provided with a tap 940 for sampling water.

The probe-holding module 100 of the first type is configured to house a flow meter dedicated to the measure of volumetric flow rate, optionally indicated by a suitably calibrated graduated scale present on the front wall of the probe-holding module 100. Making reference to Figure 10, it may be observed that a float 1100, optionally of plastic material, is advantageously inserted into the longitudinal central duct 110, that is provided with a permanent magnet 1110 configured to interact with a Reed switch 1120 housed in the rear hollow seat 150 of the probe-holding module 100; in particular, the Reed switch 1120 is actuated by the permanent magnet 1110 (that is inserted into the float 1100) when the volumetric flow rate has a value in a predetermined range, optionally ranging from 60 l/h to 80 l/h.

The modular probe-holder is usually part of a system wherein an electronic control unit is connected to the sensing probes housed in the modular probe-holder and to possible further components and apparatuses (e.g. at least one dosing pump). In the case where the measurements of volumetric flow rate carried out by the Reed switch 1120 sense that the volumetric flow rate at the inlet of the modular probe-holder is not within a predetermined range (optionally ranging from 60 l/h to 80 l/h), the electronic control unit disables all the components and apparatuses to which it is connected and which are controlled by the same electronic unit (e.g. the dosing pumps) and it generates a corresponding alarm signal. If the modular probe-holder also comprises the fifth probe-holding module 500 that houses an amperometric sensor 970, this allows to take account of the detections carried out by such amperometric sensor 970 outside the predetermined range of volumetric flow rate, which detections are not reliable because the percentage errors of the measurements are not tolerable.

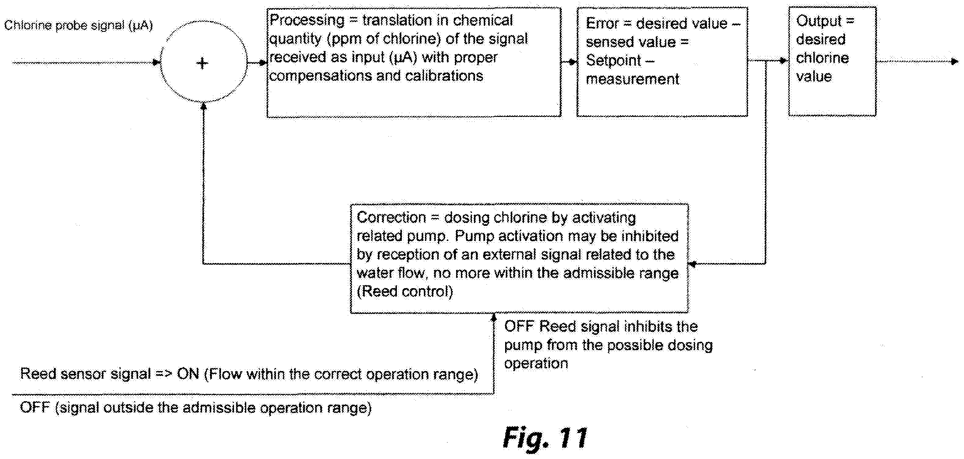

In particular, the electronic control unit may carry out a closed-loop control of the components and apparatuses connected to it. By way of example, a block diagram (immediately comprehensible to those skilled in the art) is reported in Figure 11 that schematically shows such closed-loop control wherein the amperometric sensor 970 measures the level of chlorine in water and, when such level is lower than a desired value (indicated as Setpoint), it activates a chlorine dosing pump for introducing chlorine. However, in the case where the volumetric flow rate is outside the predetermined range wherein the measure carried out by the amperometric sensor is sufficiently reliable (i.e. the percentage errors of the measurement are higher than a threshold value), then the electronic control unit disables all the components and apparatuses which it controls and it generates a corresponding alarm signal.

Other embodiments of the modular probe-holder according to the invention may comprise, instead of the Reed switch 1120, a different proximity sensor interacting with the float 1100, such as for instance a Hall effect sensor, that senses when the volumetric flow rate at the inlet of the modular probe-holder is not within a predetermined range even different from that ranging from 60 l/h to 80 l/h, optionally a predetermined range ranging from 60 l/h to 100 l/h.

It should be understood that the specific arrangement based on the interaction of the float 1100 with the Reed switch 1120 (or with a different proximity sensor, such as for instance a Hall effect sensor), and the related closed-loop control, illustrated with reference to Figures 10 and 11, are also applicable to probe-holders different from the modular probe-holder according to the invention.

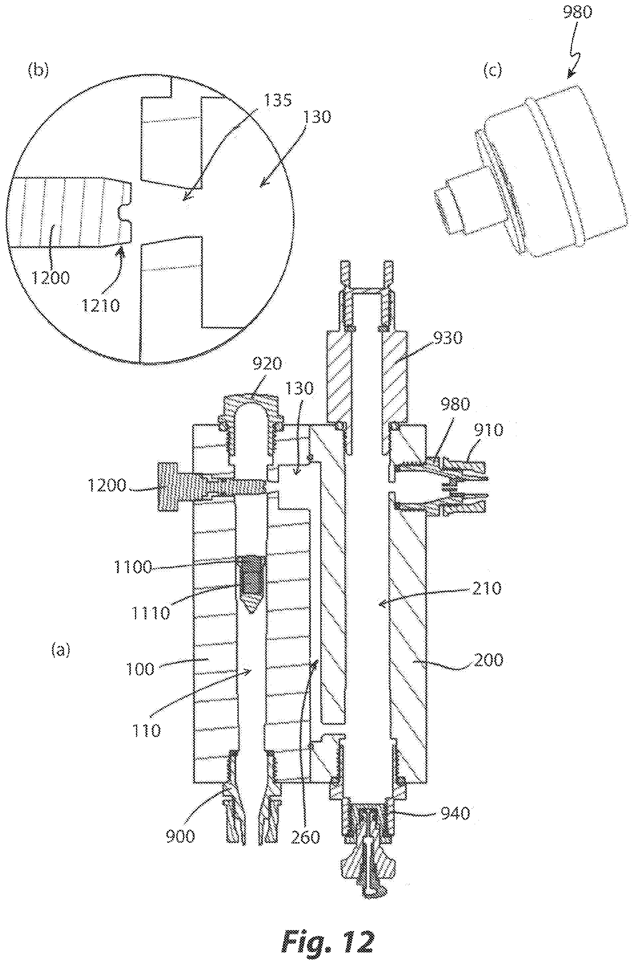

Making reference to Figure 12a, it may be observed that the probe-holding module 100 of the first type is configured to allow an adjustment of the volumetric flow rate of the fluid flowing in the probe-holder, by operating a regulating tap threaded valve 1200 housed in the upper left side hollow seat 140 of the probe-holding module 100. The valve 1200 is optionally made with a thread with fine pitch, having optionally diameter equal to 10 mm and pitch not larger than 1 mm, so as to allow a fine adjustment of the flow rate down to a complete closure of the section of the upper right transverse duct 135 where the fluid flows for reaching the upper right side outlet 130.

As shown in greater detail in Figure 12b, the valve 1200 has a conical tip 1210 having generatrices inclined by an angle ranging from 8° to 10° with respect to the longitudinal axis of symmetry of the valve 1200, so as to minimise the load losses related to the shrinkage of the section of the upper right transverse duct 135; the upper right transverse duct 135 is also conical with generatrices inclined by an angle equal to the inclination angle of the generatrices of the conical tip 1210, whereby the generatrices of the upper right transverse duct 135 are parallel to those of the conical tip 1210.

It should be understood that the specific arrangement for adjusting the volumetric flow rate based on the regulating tap threaded valve 1200, illustrated with reference to Figure 12, is also applicable to probe-holders different from the modular probe-holder according to the invention.

Moreover, as shown in greater detail in Figure 12c, in order to guarantee that the volumetric flow rate does not exceed a threshold maximum value, optionally not larger than 100 l/h (e.g. in the case where the proximity sensor is a Hall effect sensor), more optionally equal to 80 l/h (e.g. in the case where the proximity sensor is a Reed switch), it is possible to insert by means of an interference fit a flow rate regulator 980 in the outlet tube-holder 910 of to be coupled to the upper right side outlet of the final probe-holding module of the modular probe-holder.

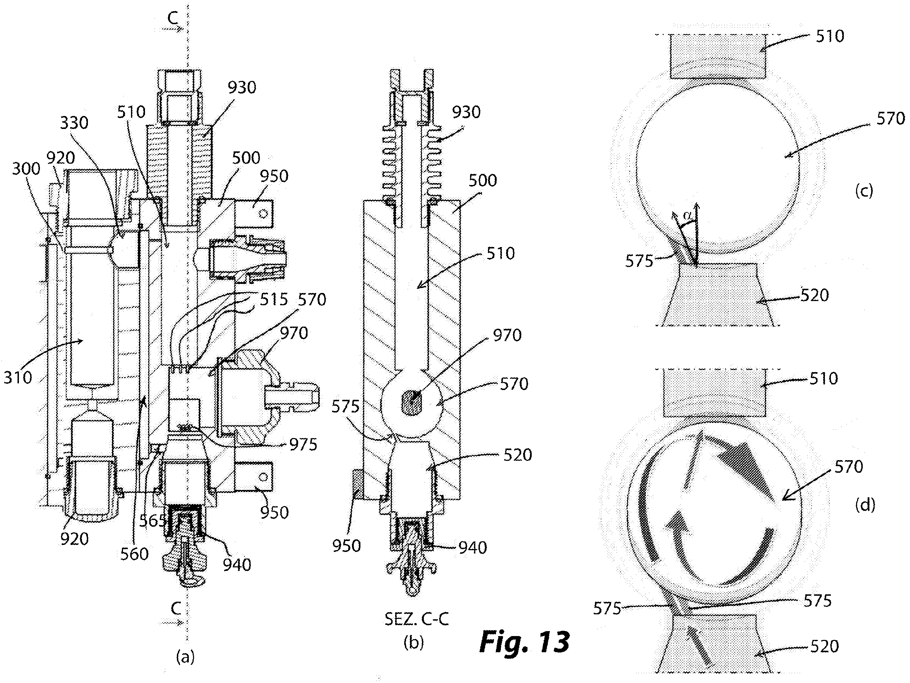

As shown in Figures 13a and 13b, the probe-holding module 500 of the fifth type is configured to house a horizontal amperometric sensor 970 in the lower right side hollow seat 570, having a circular or ellipsoidal transverse section (as shown in Figure 13b), that inferiorly communicates with the lower threaded mouth 520 through a plurality of inner tubular ducts 575 (in the preferred embodiment of Figure 13, such inner tubular ducts 575 are seven, arranged along two rows visible in Figures 13c and 13d as aligned sideways, of which the outlet mouths 975 are visible in Figure 13a in the lower right side hollow seat 570), the diameter of which (optionally equal to 2 mm) is significantly lower than its length (optionally equal to 12 mm).

The probe-holding module 500 is provided with an upper left transverse duct 580 (that is optionally tubular) that, in correspondence with the upper right side threaded outlet 330 of the adjacent module 300, puts in communication the upper portion of the groove 560 on the left side wall with the upper portion of the longitudinal duct 510. Such upper left transverse duct 580 allows the air present in the fluid entering the groove 560 on the left side wall to be eliminated by the Venturi effect; in other words, the upper left transverse duct 580 operates as by-pass for air mixed with the fluid entering the fifth probe-holding module 500. In this way, the upper left transverse duct 580 achieves a degassing of the fluid entering the fifth probe-holding module 500 that is particularly important for avoiding malfunctions of the amperometric sensor 970; in particular, the amount of air mixed with the fluid is larger when the fluid starts to flow in the modular probe-holder.

As shown in greater detail in Figures 13c and 13d, the water flow (schematically represented in Figure 13d by the arrows) in the probe-holding module 500 is channelled from the bottom upwards in the lower right side hollow seat 570 through the inner tubular ducts 575 each having an inclination angle a, with respect to the transverse plane crossing the right and left side walls of the probe-holding module 500, optionally ranging from 20° to 30°, more optionally equal to 25°. This channelling, by accelerating the flow, allows the solute to be agitated and further guarantees the entrainment of the balls (optionally of plastics, glass or ceramic, not shown) housed in the lower right side hollow seat 570 for cleaning the electrode of the amperometric sensor. Moreover, the lower right side hollow seat 570 communicates superiorly with the longitudinal duct 510, through a plurality (Figure 13 shows three) of planar ducts 515 (i.e. having a dimension much lower than the other two), oriented parallel to the longitudinal axis of the longitudinal duct 510.

The upper left transverse duct 580, as well as the arrangement of the inner tubular ducts 575 and planar ducts 515, which are also adoptable independently from one another, are not features essential to the modular probe-holder according to the invention.

It should be understood that the specific arrangement of the upper left transverse duct 580, illustrated with reference to Figure 13a, is applicable (also independently from the arrangement of the inner tubular ducts 575 and planar ducts 515) also to probe-holders different from the modular probe-holder according to the invention.

Similarly, it should be understood that the specific arrangement of the inner tubular ducts 575 and planar ducts 515, illustrated with reference to Figure 13, is applicable (also independently from the arrangement of the upper left transverse duct 580) also to probe-holders different from the modular probe-holder according to the invention.

The preferred embodiments of this invention have been described and a number of variations have been suggested hereinbefore, but it should be understood that those skilled in the art can make other variations and changes, without so departing from the scope of protection thereof, as defined by the attached claims.

## Claims

1. Modular probe-holder configured to house sensing probes for sensing physico-chemical parameters of a fluid, **characterised in that** it comprises two or more probe-holding modules (100; 200; 300; 400; 500), each one configured to house at least one sensing probe, which are laterally coupled in pairs so that each pair of laterally coupled probe-holding modules (100; 200; 300; 400; 500) comprises a first probe-holding module (100; 200; 300; 400), including a first duct (110; 210; 310; 410) connected to a first inlet (120; 260; 360; 460) and to a first outlet (130; 230; 330; 430), and a second probe-holding module (200; 300; 400; 500), including a second duct (210; 310; 410; 510) connected to a second inlet (260; 360; 460; 560) and to a second outlet (230; 330; 430; 530), the first outlet (130; 230; 330; 430) being arranged on a first side wall of the first probe-holding module (100; 200; 300; 400) and the second inlet (260; 360; 460; 560) being arranged on a second side wall of the second probe-holding module (200; 300; 400; 500), whereby the first side wall of the first probe-holding module (100; 200; 300; 400) is coupled to the second side wall of the second probe-holding module (200; 300; 400; 500) so that the first outlet (130; 230; 330; 430) communicates with the second inlet (260; 360; 460; 560), each pair of laterally coupled probe-holding modules (100; 200; 300; 400; 500) being configured to receive a flow of a fluid from the first inlet (120; 260; 360; 460) to the second outlet (230; 330; 430; 530), the first and second probe-holding modules (100; 200; 300; 400; 500) being coupled through fastening mechanical means (700; 750) inserted into at least one pair of facing seats, wherein a first seat (660; 680) is arranged on the first side wall of the first probe-holding module (100; 200; 300; 400) and a second seat (650; 670) is arranged on the second side wall of the second probe-holding module (200; 300; 400; 500), at least one elastic seal (810) being inserted into at least one seal groove (800), with which at least one from the first and second side walls is provided, said at least one seal groove (800) being arranged for surrounding the first outlet (130; 230; 330; 430) and the second inlet (260; 360; 460; 560), whereby said at least one elastic seal (810) seals the coupling of the first side wall of the first probe-holding module (100; 200; 300; 400) to the second side wall of the second probe-holding module (200; 300; 400; 500).

2. Modular probe-holder according to claim 1, **characterised in that** said fastening mechanical means comprises at least one tie (700) inserted into said at least one pair of facing seats, the first seat (660; 680) communicating with a first threaded hole (610; 630; 15), that has a longitudinal axis and that is arranged on a front or rear wall of the first probe-holding module (100; 200; 300; 400), into which a first grub screw (710) is screwed, the second seat (650; 670) communicating with a second threaded hole (600; 620; 20), that has a longitudinal axis and that is arranged on a front or rear wall of the second probe-holding module (200; 300; 400; 500), into which a second grub screw (710) is screwed, said at least one tie (700) comprising a first and a second holes (701) having respective longitudinal axes and configured to receive a tip (711) of the first and second grub screws (710), respectively, the tip (711) of each one of the first and second grub screws (710) having a conical lateral surface, optionally with apex angle equal to 90°, the first and second holes (701) of said at least one tie (700) having a support inclined surface corresponding to the conical lateral surface of the tip (711) of each one of the first and second grub screws (710), a distance DA between the longitudinal axes of the first and second threaded holes (610, 600; 630, 620; 15, 20) being larger than the distance DB between the longitudinal axes of the first and second holes (701) of said at least one tie (700), optionally by an amount ranging from 2% to 3%, more optionally by an amount equal to 2,5%, whereby when the tips (711) of the first and second grub screws (710) are received in the first and second holes (701) of said at least one tie (700) the first and second probe-holding modules (100; 200; 300; 400; 500) are coupled to each other.

3. Modular probe-holder according to claim 1, **characterised in that** said fastening mechanical means comprises at least one threaded stud (750) screwed into said at least one pair of facing seats, whereby the first seat (660; 680) and the second seat (650; 670) are threaded.

4. Modular probe-holder according to any one of the preceding claims, **characterised in that** said at least one elastic seal (810) has circular cross-section having diameter w and said at least one seal groove (800) has rectangular cross-section, having width w equal to the diameter w and depth p, wherein p ranges from 65% to 75% of w, p being optionally equal to 70% of w, said at least one elastic seal (810) being optionally made of fluoroelastomer.

5. Modular probe-holder according to any one of the preceding claims, **characterised in that** the first duct (110; 210; 310; 410) and the second duct (210; 310; 410; 510) are vertical, the first inlet (120; 260; 360; 460), the first outlet (130; 230; 330; 430), the second inlet (260; 360; 460; 560) and the second outlet (230; 330; 430; 530) being arranged so that the first duct (110; 210; 310; 410) and the second duct (210; 310; 410; 510) are configured to receive said flow of the fluid from the bottom upwards.

6. Modular probe-holder according to claim 5, **characterised in that** the first outlet (130; 230; 330; 430) is superiorly arranged on the first side wall of the first probe-holding module (100; 200; 300; 400) and the second inlet comprises a groove (260; 360; 460; 560) on the second right side wall of the second probe-holding module (200; 300; 400; 500) that communicates with the second duct (210; 310; 410; 510) through a lower transverse duct (265; 365; 465; 565).

7. Modular probe-holder according to any one of the preceding claims, **characterised in that** each one of said two or more probe-holding modules (100; 200; 300; 400; 500) is a type of probe-holding module (100; 200; 300; 400; 500) selected from a group comprising two or more types of probe-holding modules (100; 200; 300; 400; 500) which are different and capable to be coupled to each other, optionally made of a transparent plastic material, more optionally made of poly(methyl methacrylate).

8. Probe-holding module (100; 200; 300; 400; 500) for use in the modular probe-holder according to any one of claims 1 to 7, configured to house at least one sensing probe, that includes a duct (110; 210; 310; 410; 510) connected to an inlet (120; 260; 360; 460; 560) and to an outlet (130; 230; 330; 430; 530), the probe-holding module (100; 200; 300; 400; 500) being configured to receive a flow of a fluid from the inlet (120; 260; 360; 460; 560) to the outlet (130; 230; 330; 430; 530) and having a first side wall and a second side wall, at least one from the inlet (120; 260; 360; 460; 560) and the outlet (130; 230; 330; 430; 530) being on one between the first and second side walls, the probe-holding module (100; 200; 300; 400; 500) being **characterised in that** it is provided, on at least one between the first and second side walls, with at least one seat (650; 660; 670; 680) configured to receive fastening mechanical means (700; 750).

9. Probe-holding module (100; 200; 300; 400; 500) according to claim 8, **characterised in that** said at least one seat (650; 660; 670; 680) is configured to partially receive at least one tie (700), said at least one seat (650; 660; 670; 680) communicating with at least one threaded hole (610; 630; 15) arranged on a front or rear wall of the probe-holding module (100; 200; 300; 400; 500) and configured to receive at least one grub screw (710).

10. Probe-holding module (100; 200; 300; 400; 500) according to claim 8, **characterised in that** said at least one seat (650; 660; 670; 680) is screwed and it is configured to receive at least one threaded stud (750).

11. Probe-holding module (100; 200; 300; 400; 500) according to any one of claims 8 to 10, **characterised in that** it is further provided, on at least one between the first and second side walls on which the inlet (120; 260; 360; 460; 560) or the outlet (130; 230; 330; 430; 530) is present, with at least one seal groove (800) configured to receive at least one elastic seal (810) and arranged for surrounding the inlet (120; 260; 360; 460; 560) or the outlet (130; 230; 330; 430; 530) present on the side wall provided with said at least one seal groove (800), said at least one seal groove (800) optionally having rectangular cross-section.

12. Probe-holding module (100) according to any one of claims 8 to 11, **characterised in that** it is configured to house a flow meter (1100, 1110, 1120), optionally comprising a rear hollow seat (150) configured to house a proximity sensor (1120), more optionally a Reed switch or a Hall effect sensor, the duct of the probe-holding module (100) being a vertical duct (110) configured to house a float (1100) interacting with a proximity sensor (1120) when the latter is housed in the rear hollow seat (150), the outlet (130) of the probe-holding module (100) being arranged on one between the first and second side walls, the inlet of the probe-holding module (100) being a lower mouth (120) and the outlet (130) of the probe-holding module (100) being superiorly arranged on one between the first and second side walls, the outlet (130) of the probe-holding module (100) being connected to the vertical duct (110) through an upper transverse duct (135), the probe-holding module (100) having an upper side hollow seat (140) wherein a regulating tap threaded valve (1200) having a conical tip (1210), the generatrices of which are inclined by an angle optionally ranging from 8° to 10° with respect to a longitudinal axis of the valve (1200), the upper transverse duct (135) being conical with generatrices inclined by an angle equal to the inclination angle of the generatrices of the conical tip (1210) of the valve (1200), the valve (1200) optionally having a thread with pitch not larger than 1 mm.

13. Probe-holding module (500) according to any one of claims 8 to 11, **characterised in that** the duct of the probe-holding module (500) is a vertical duct (510), the probe-holding module (500) being configured to house an amperometric sensor (970) in a lower hollow seat (570) superiorly communicating with the vertical duct (510), the inlet (530) of the probe-holding module (500) comprising a groove (560) on an inlet side wall selected between the first and second side walls, the groove (560) communicating with the vertical duct (510) through a lower transverse duct (565), the outlet (530) of the probe-holding module (500) being superiorly arranged on an outlet side wall selected between the first and second side walls differing from the inlet side wall, the probe-holding module (500) being further provided with an upper transverse, optionally tubular, duct (580) that puts in communication an upper portion of the groove (560) with an upper portion of the vertical duct (510).

14. Probe-holding module (500) according to claim 13, **characterised in that** the lower transverse duct (565) is connected between the groove (560) and a lower mouth (520) communicating externally, the lower hollow seat (570) being configured to house a plurality of balls, the lower hollow seat (570) communicating with the lower mouth (520) through a plurality of inner tubular ducts (575) inclined with respect to a transverse plane crossing the first and second side walls, an inclination angle α of each inner tubular duct (575) with respect to said transverse plane optionally ranging from 20° to 30°, more optionally being equal to 25°, the lower hollow seat (570) superiorly communicating with the vertical duct (510) through one or more planar ducts (515) oriented parallel to a longitudinal axis of the vertical duct (510).

15. Probe-holding module (500) according to any one of claims 8 to 14, **characterised in that** it is made of transparent plastic material, optionally poly(methyl methacrylate), the module being optionally provided on a rear wall with at least one pair of holes (850, 860), each hole (850, 860) being configured to receive a screw for fastening one or more rear brackets.

## Patentansprüche

1. Modularer Sondenhalter, der konfiguriert ist, um Sonden zum Erfassen physikalischchemischer Parameter eines Fluids aufzunehmen, **dadurch gekennzeichnet, dass** er zwei oder mehrere Sondenhalterungsmodule (100; 200; 300; 400; 500) umfasst, die jeweils konfiguriert sind, um mindestens eine Sonde aufzunehmen, die seitlich paarweise gekoppelt sind, so dass jedes Paar seitlich gekoppelter Sondenhalterungsmodule (100; 200; 300; 400; 500) ein erstes Sondenhaltemodul (100; 200; 300; 400), das einen ersten Kanal (110; 210; 310; 410) umfasst, der mit einem ersten Eingang (120; 260; 360; 460) und einem ersten Ausgang (130; 230; 330; 430) verbunden ist, und ein zweites Sondenhaltemodul (200; 300; 400; 500), umfassend einen zweiten Kanal (210; 310; 410; 510), der mit einem zweiten Einlass (260; 360; 460; 560) und einem zweiten Auslass (230; 330; 430; 530) verbunden ist, umfasst,
wobei der erste Auslass (130; 230; 330; 430) an einer ersten Seitenwand des ersten Sondenhaltemoduls (100; 200; 300; 400) und der zweite Einlass (260, 360; 460; 560) an einer zweiten Seitenwand des zweiten Sondenhaltemoduls (200; 300; 400; 500) angeordnet ist,
wobei die erste Seitenwand des ersten Sondenhaltemoduls (100; 200; 300; 400; 400) mit der zweiten Seitenwand des zweiten Sondenhaltemoduls (200; 300; 400; 500) gekoppelt ist, so dass der erste Ausgang (130; 230; 330; 430) mit dem zweiten Einlass (260; 360; 460; 560) in Verbindung steht, wobei jedes Paar seitlich gekoppelter Sondenhaltemodule (100; 200; 300; 400; 500) konfiguriert ist, um einen Fluidstrom vom ersten Einlass (120; 260; 360; 460) zum zweiten Auslass (230; 330; 430, 530) aufzunehmen, wobei das erste und das zweite Sondenhaltemodul (100; 200; 300; 400; 500) durch mechanische Befestigungsmittel (700; 750) gekoppelt sind, die in mindestens ein Paar von gegenüberliegenden Sitzen eingesetzt sind, wobei ein erster Sitz (660; 680) an der ersten Seitenwand des ersten Sondenhaltemoduls (100; 200; 300; 400) und ein zweiter Sitz (650; 670) an der zweiten Seitenwand des zweiten Sondenhaltemoduls (200; 300; 400; 500) angeordnet ist, wobei mindestens eine elastische Dichtung (810) in mindestens eine Dichtungsnut (800) eingesetzt ist, mit der mindestens eine von der ersten und zweiten Seitenwand versehen ist, wobei die mindestens eine Dichtungsnut (800) angeordnet ist, um den ersten Auslass (130; 230; 330; 430) und den zweiten Einlass (260; 360; 460; 560) zu umgeben, wobei die mindestens eine elastische Dichtung (810) die Kupplung der ersten Seitenwand des ersten Sondenhaltemoduls (100; 200; 300; 400) mit der zweiten Seitenwand des zweiten Sondenhaltemoduls (200; 300; 400; 500) dichtet.

2. Modularer Sondenhalter nach Anspruch 1, **dadurch gekennzeichnet, dass** die mechanischen Befestigungsmittel mindestens eine Verbindung (700) umfassen, die in das mindestens eine Paar von gegenüberliegenden Sitzen eingesetzt ist, wobei der erste Sitz (660; 680) mit einem ersten Gewindeloch (610; 630; 15) verbunden ist, das eine Längsachse aufweist und an einer Vorder- oder Rückwand des ersten Sondenhaltemoduls (100; 200; 300; 400) angeordnet ist, in die eine erste Madenschraube (710) eingeschraubt ist, wobei der zweite Sitz (650; 670) mit einem zweiten Gewindeloch (600; 620; 20) in Verbindung steht, das eine Längsachse aufweist und das an einer Vorder- oder Rückwand des zweiten Sondenhaltemoduls (200; 300; 400; 500) angeordnet ist, in die ein zweiter Gewindestift (710) eingeschraubt ist, wobei die mindestens eine Verbindung (700) ein erstes und ein zweites Loch (701) mit entsprechenden Längsachsen umfasst und zur Aufnahme einer Spitze (711) der ersten bzw. zweiten Gewindestifte (710) konfiguriert ist, wobei die Spitze (711) jeder der ersten und zweiten Gewindestifte (710) eine konische Seitenfläche aufweist, optional mit einem Scheitelwinkel von gleich 90°, wobei die ersten und zweiten Löcher (701) des mindestens einen Binders (700) eine der konischen Seitenfläche der Spitze (711) jeder der ersten und zweiten Gewindestifte (710) entsprechende Stützschrägfläche aufweisen, einen Abstand DA zwischen den Längsachsen der ersten und zweiten Gewindebohrungen (610, 600; 630, 620; 15, 20) größer als der Abstand DB zwischen den Längsachsen der ersten und zweiten Löcher (701) des mindestens einen Binders (700) ist, gegebenenfalls um einen Betrag im Bereich von 2% bis 3%, gegebenenfalls um einen Betrag im Bereich von 2,5%, wobei, wenn die Spitzen (711) der ersten und zweiten Gewindestifte (710) in den ersten und zweiten Löchern (701) des mindestens einen Binders (700) aufgenommen werden, die ersten und zweiten Sondenhaltemodule (100; 200; 300; 400; 500) miteinander verbunden sind.

3. Modularer Sondenhalter nach Anspruch 1, **dadurch gekennzeichnet, dass** die mechanischen Befestigungsmittel mindestens einen Gewindebolzen (750) umfassen, der in das mindestens eine Paar von gegenüberliegenden Sitzen eingeschraubt ist, wobei der erste Sitz (660; 680) und der zweite Sitz (650; 670) mit Gewinde versehen sind.

4. Modularer Sondenhalter nach einem der vorstehenden Ansprüche, **dadurch gekennzeichnet, dass** die mindestens eine elastische Dichtung (810) einen kreisförmigen Querschnitt mit dem Durchmesser w aufweist und die mindestens eine Dichtungsnut (800) einen rechteckigen Querschnitt mit der Breite w gleich dem Durchmesser w und der Tiefe p aufweist, wobei p im Bereich von 65 % bis 75 % von w liegt, wobei p optional gleich 70 % von w ist und die mindestens eine elastische Dichtung (810) optional aus Fluorelastomer besteht.

5. Modularer Sondenhalter nach einem der vorhergehenden Ansprüche, **dadurch gekennzeichnet, dass** der erste Kanal (110; 210; 310; 410) und der zweite Kanal (210; 310; 410; 510) vertikal sind, der erste Eingang (120; 260; 360; 460), der erste Ausgang (130; 230; 330; 430), der zweite Eingang (260; 360; 460; 560) und der zweite Ausgang (230; 330; 430; 530) so angeordnet sind, dass der erste Kanal (110; 210; 310; 410) und der zweite Kanal (210; 310; 410; 510) konfiguriert sind, um den Strom des Fluids von unten nach oben aufzunehmen.

6. Modularer Sondenhalter nach Anspruch 5, **dadurch gekennzeichnet, dass** der erste Auslass (130; 230; 330; 430) an der ersten Seitenwand des ersten Sondenhaltemoduls (100; 200; 300; 400) und der zweite Einlass eine Nut (260; 360; 460; 560) an der zweiten rechten Seitenwand des zweiten Sondenhaltemoduls (200; 300; 400; 500) aufweist, die mit dem zweiten Kanal (210; 310; 410; 510) durch einen unteren Querkanal (265; 365; 465; 565) verbunden ist.

7. Modularer Sondenhalter nach einem der vorstehenden Ansprüche, **dadurch gekennzeichnet, dass** jedes der beiden oder mehreren Sondenhaltemodule (100; 200; 300; 400; 500) eine Art Sondenhaltemodul (100; 200; 300; 400; 500) ist, ausgewählt aus einer Gruppe, die zwei oder mehr Arten von Sondenhaltemodulen (100; 200; 300; 400; 500) umfasst, die unterschiedlich und miteinander koppelbar sind, optional aus einem transparenten Kunststoffmaterial, optional mehr aus Poly(methylmethacrylat).

8. Sondenhaltemodul (100; 200; 300; 400; 500) zur Verwendung in dem modularen Sondenhalter nach einem der Ansprüche 1 bis 7, konfiguriert zur Aufnahme mindestens einer Messsonde, das einen Kanal (110; 210; 310; 410; 510) beinhaltet, der mit einem Einlass (120; 260; 360; 460; 560) und einem Auslass (130; 230; 330; 430; 530) verbunden ist, wobei das Sondenhaltemodul (100; 200; 300; 400; 500) konfiguriert ist, um einen Fluidstrom vom Einlass (120; 260; 360; 460; 560) zum Auslass (130; 230; 330; 430; 530) aufzunehmen und umfassend eine erste Seitenwand und eine zweite Seitenwand, wobei mindestens eine aus dem Einlass (120; 260; 360; 460; 560) und dem Auslass (130; 230; 330; 430; 530) auf einer zwischen der ersten und zweiten Seitenwand angeordnet ist, wobei das Sondenhaltemodul (100; 200; 300; 400; 500) **dadurch gekennzeichnet ist, dass** es an mindestens einer zwischen der ersten und zweiten Seitenwand mit mindestens einem Sitz (650; 660; 670; 680) versehen ist, der zur Aufnahme von mechanischen Befestigungsmitteln (700; 750) ausgebildet ist.

9. Sondenhaltemodul (100; 200; 300; 400; 500) nach Anspruch 8, **dadurch gekennzeichnet, dass** der mindestens eine Sitz (650; 660; 670; 680) konfiguriert ist, um mindestens eine Verbindung (700) teilweise aufzunehmen, wobei der mindestens eine Sitz (650; 660; 670; 680) mit mindestens einem Gewindeloch (610; 630; 15) verbunden ist, das an einer Vorder- oder Rückwand des Sondenhaltemoduls (100; 200; 300; 400; 500) angeordnet und konfiguriert ist, um mindestens eine Madenschraube (710) aufzunehmen.

10. Sondenhaltemodul (100; 200; 300; 400; 500) nach Anspruch 8, **dadurch gekennzeichnet, dass** der mindestens eine Sitz (650; 660; 670; 680) verschraubt ist und zur Aufnahme von mindestens einem Gewindebolzen (750) ausgebildet ist.

11. Sondenhaltemodul (100; 200; 300; 400; 500) nach einem der Ansprüche 8 bis 10, **dadurch gekennzeichnet, dass** es ferner an mindestens einer zwischen der ersten und zweiten Seitenwand, an der der Einlass (120; 260; 360; 460; 560) oder der Auslass (130; 230; 330; 430; 530) vorhanden ist, mit mindestens einer Dichtungsnut (800) versehen ist, die zur Aufnahme mindestens einer elastischen Dichtung (810) ausgebildet und zum Umgeben des Einlasses (120; 260; 360; 460; 560) oder des Auslasses (130; 230; 330; 430; 530) an der Seitenwand, die mit der mindestens einen Dichtungsnut (800) versehen ist, angeordnet ist, wobei die mindestens eine Dichtungsnut (800) wahlweise rechteckigen Querschnitt aufweist.

12. Sondenhaltemodul (100) nach einem der Ansprüche 8 bis 11, **dadurch gekennzeichnet, dass** es zur Aufnahme eines Durchflussmessers (1100, 1110, 1120) konfiguriert ist, der optional einen hinteren Hohlsitz (150) zur Aufnahme eines Näherungssensors (1120), optional einen Reedschalter oder einen Halleffekt-Sensor umfasst, wobei der Kanal des Sondenhaltemoduls (100) ein vertikaler Kanal (110) ist, der zur Aufnahme eines Schwimmers (1100) konfiguriert ist, der mit einem Näherungssensor (1120) zusammenwirkt, wenn dieser in dem hinteren Hohlsitz (150) untergebracht ist, der Auslass (130) des Sondenhaltemoduls (100) zwischen der ersten und zweiten Seitenwand angeordnet ist, wobei der Einlass des Sondenhaltemoduls (100) eine untere Öffnung (120) und der Auslass (130) des Sondenhaltemoduls (100) zwischen der ersten und der zweiten Seitenwand überlegen angeordnet ist, der Auslass (130) des Sondenhaltemoduls (100) mit dem vertikalen Kanal (110) durch einen oberen Querkanal (135) verbunden ist, wobei das Sondenhaltemodul (100) einen oberen seitlichen Hohlsitz (140) aufweist, wobei ein Regelgewindeventil (1200) mit einer konischen Spitze (1210), deren Erzeugungen um einen Winkel geneigt sind, der gegebenenfalls von 8° bis 10° in Bezug auf eine Längsachse des Ventils (1200) reicht, wobei der obere Querkanal (135) konisch ist und die Erzeugungen um einen Winkel geneigt sind, der dem Neigungswinkel der Erzeugungen der konischen Spitze (1210) des Ventils (1200) entspricht, wobei das Ventil (1200) optional ein Gewinde mit einer Steigung von nicht mehr als 1 mm aufweist.

13. Sondenhaltemodul (500) nach einem der Ansprüche 8 bis 11, **dadurch gekennzeichnet, dass** der Kanal des Sondenhaltemoduls (500) ein vertikaler Kanal (510) ist, wobei das Sondenhaltemodul (500) konfiguriert ist, um einen amperometrischen Sensor (970) in einem unteren Hohlsitz (570) aufzunehmen, der mit dem vertikalen Kanal (510) besser verbunden ist, wobei der Einlass (530) des Sondenhaltemoduls (500) eine Nut (560) an einer Einlassseitenwand aufweist, die zwischen der ersten und zweiten Seitenwand ausgewählt ist, die Nut (560), die mit dem vertikalen Kanal (510) durch einen unteren Querkanal (565) in Verbindung steht, wobei der Auslass (530) des Sondenhaltemoduls (500) übergeordnet auf einer Auslassseitenwand angeordnet ist, die zwischen der ersten und der zweiten Seitenwand ausgewählt ist, die sich von der Einlassseitenwand unterscheiden, wobei das Sondenhaltemodul (500) ferner mit einem oberen transversalen, optional rohrförmigen Kanal (580) versehen ist, der einen oberen Abschnitt der Nut (560) mit einem oberen Abschnitt des vertikalen Kanals (510) in Verbindung setzt.

14. Sondenhaltemodul (500) nach Anspruch 13, **dadurch gekennzeichnet, dass** der untere Querkanal (565) zwischen der Nut (560) und einer nach außen gerichteten unteren Öffnung (520) verbunden ist, wobei der untere Hohlsitz (570) zur Aufnahme einer Vielzahl von Kugeln ausgebildet ist, wobei der untere Hohlsitz (570) mit der unteren Öffnung (520) durch eine Vielzahl von inneren Rohrkanälen (575) verbunden ist, die in Bezug auf eine die erste und zweite Seitenwand kreuzende Querebene geneigt sind, einen Neigungswinkel α jedes inneren Rohrkanals (575) in Bezug auf die Transversalebene, der gegebenenfalls von 20° bis 30° reicht, wobei der untere Hohlsitz (570) optional gleich 25° ist, wobei der untere Hohlsitz (570) über einen oder mehrere planare Kanäle (515), die parallel zu einer Längsachse des vertikalen Kanals (510) ausgerichtet sind, mit dem vertikalen Kanal (510) in Verbindung steht.

15. Sondenhaltemodul (500) nach einem der Ansprüche 8 bis 14, **dadurch gekennzeichnet, dass** es aus transparentem Kunststoffmaterial, gegebenenfalls Poly(methylmethacrylat), hergestellt ist, wobei das Modul optional an einer Rückwand mit mindestens einem Lochpaar (850, 860) versehen ist, wobei jedes Loch (850, 860) zur Aufnahme einer Schraube zur Befestigung einer oder mehrerer hinterer Halterungen ausgebildet ist.

## Revendications

1. Porte-sonde modulaire configuré pour loger des sondes de détection pour détecter des paramètres physico-chimiques d'un fluide, **caractérisé en ce qu'**il comprend au moins deux modules porte-sonde (100 ; 200 ; 300 ; 400 ; 500), chacun étant configuré pour loger au moins une sonde de détection, qui sont latéralement couplés par paires de sorte que chaque paire de modules porte-sonde latéralement couplés (100 ; 200 ; 300 ; 400 ; 500) comprenne un premier module porte-sonde (100 ; 200 ; 300 ; 400), incluant une première conduite (110 ; 210 ; 310 ; 410) reliée à une première entrée (120 ; 260 ; 360 ; 460) et à une première sortie (130 ; 230 ; 330 ; 430), et un second module porte-sonde (200 ; 300 ; 400 ; 500), incluant une seconde conduite (210 ; 310 ; 410 ; 510) reliée à une seconde entrée (260 ; 360 ; 460 ; 560) et à une seconde sortie (230 ; 330 ; 430 ; 530), la première sortie (130 ; 230 ; 330 ; 430) étant agencée sur une première paroi latérale du premier module porte-sonde (100 ; 200 ; 300 ; 400) et la seconde entrée (260 ; 360 ; 460 ; 560) étant agencée sur une seconde paroi latérale du second module porte-sonde (200 ; 300 ; 400 ; 500), moyennant quoi la première paroi latérale du premier module porte-sonde (100 ; 200 ; 300 ; 400) est couplée à la seconde paroi latérale du second module porte-sonde (200 ; 300 ; 400 ; 500) de sorte que la première sortie (130 ; 230 ; 330 ; 430) communique avec la seconde entrée (260 ; 360 ; 460 ; 560), chaque paire de modules porte-sonde latéralement couplés (100 ; 200 ; 300 ; 400 ; 500) étant configurée pour recevoir un écoulement d'un fluide de la première entrée (120 ; 260 ; 360 ; 460) à la seconde sortie (230 ; 330 ; 430 ; 530), les premier et second modules porte-sonde (100 ; 200 ; 300 ; 400 ; 500) étant couplés par l'intermédiaire d'un moyen mécanique de fixation (700 ; 750) inséré dans au moins une paire de sièges se faisant face, dans lequel un premier siège (660 ; 680) est agencé sur la première paroi latérale du premier module porte-sonde (100 ; 200 ; 300 ; 400) et un second siège (650 ; 670) est agencé sur la seconde paroi latérale du second module porte-sonde (200 ; 300 ; 400 ; 500), au moins un joint d'étanchéité élastique (810) étant inséré dans au moins une rainure de joint d'étanchéité (800), dont au moins l'une des première et seconde parois latérales est dotée, ladite au moins une rainure de joint d'étanchéité (800) étant agencée pour entourer la première sortie (130 ; 230 ; 330 ; 430) et la seconde entrée (260 ; 360 ; 460 ; 560), moyennant quoi ledit au moins un joint d'étanchéité élastique (810) étanchéifie le couplage de la première paroi latérale du premier module porte-sonde (100 ; 200 ; 300 ; 400) à la seconde paroi latérale du second module porte-sonde (200 ; 300 ; 400 ; 500).

2. Porte-sonde modulaire selon la revendication 1, **caractérisé en ce que** ledit moyen mécanique de fixation comprend au moins une attache (700) insérée dans ladite au moins une paire de sièges se faisant face, le premier siège (660 ; 680) communiquant avec un premier trou fileté (610 ; 630 ; 15), qui a un axe longitudinal et qui est agencé sur une paroi avant ou arrière du premier module porte-sonde (100 ; 200 ; 300 ; 400), dans laquelle une première vis sans tête (710) est vissée, le second siège (650 ; 670) communiquant avec un second trou fileté (600 ; 620 ; 20), qui a un axe longitudinal et qui est agencé sur une paroi avant ou arrière du second module porte-sonde (200 ; 300 ; 400 ; 500), dans laquelle une second vis sans tête (710) est vissée, ladite au moins une attache (700) comprenant un premier et un second trous (701) ayant des axes longitudinaux respectifs et configurés pour recevoir une pointe (711) des première et seconde vis sans tête (710), respectivement, la pointe (711) de chacune des première et seconde vis sans tête (710) ayant une surface latérale conique, éventuellement avec un angle de sommet égal à 90°, les premier et second trous (701) de ladite au moins une attache (700) ayant une surface inclinée de support correspondant à la surface latérale conique de la pointe (711) de chacune des première et seconde vis sans tête (710), une distance DA entre les axes longitudinaux des premier et second trous filetés (610, 600 ; 630, 620 ; 15, 20) étant supérieure à la distance DB entre les axes longitudinaux des premier et second trous (701) de ladite au moins une attache (700), éventuellement d'une quantité comprise entre 2 % et 3 %, encore plus éventuellement d'une quantité égale à 2,5 %, moyennant quoi lorsque les pointes (711) des première et seconde vis sans tête (710) sont reçues dans les premier et second trous (701) de ladite au moins une attache (700), les premier et second modules porte-sonde (100 ; 200 ; 300 ; 400 ; 500) sont couplés l'un à l'autre.

3. Porte-sonde modulaire selon la revendication 1, **caractérisé en ce que** ledit moyen mécanique de fixation comprend au moins un goujon fileté (750) vissé dans ladite au moins une paire de sièges se faisant face, moyennant quoi le premier siège (660 ; 680) et le second siège (650 ; 670) sont filetés.

4. Porte-sonde modulaire selon l'une quelconque des revendications précédentes, **caractérisé en ce que** ledit au moins un joint d'étanchéité élastique (810) a une section transversale circulaire ayant un diamètre w et ladite au moins une rainure de joint d'étanchéité (800) a une section transversale rectangulaire, ayant une largeur w égale au diamètre w et une profondeur p, dans lequel p est compris entre 65 % et 75 % de w, p étant éventuellement égal à 70 % de w, ledit au moins un joint d'étanchéité élastique (810) étant éventuellement constitué de fluoroélastomère.

5. Porte-sonde modulaire selon l'une quelconque des revendications précédentes, **caractérisé en ce que** la première conduite (110 ; 210 ; 310 ; 410) et la seconde conduite (210 ; 310 ; 410 ; 510) sont verticales, la première entrée (120 ; 260 ; 360 ; 460), la première sortie (130 ; 230 ; 330 ; 430), la seconde entrée (260 ; 360 ; 460 ; 560) et la seconde sortie (230 ; 330 ; 430 ; 530) étant agencées de sorte que la première conduite (110 ; 210 ; 310 ; 410) et la seconde conduite (210 ; 310 ; 410 ; 510) soient configurées pour recevoir ledit écoulement du fluide du bas vers le haut.

6. Porte-sonde modulaire selon la revendication 5, **caractérisé en ce que** la première sortie (130 ; 230 ; 330 ; 430) est agencée au niveau supérieur sur la première paroi latérale du premier module porte-sonde (100 ; 200 ; 300 ; 400) et la seconde entrée comprend une rainure (260 ; 360 ; 460 ; 560) sur la seconde paroi latérale droite du second module porte-sonde (200 ; 300 ; 400 ; 500) qui communique avec la seconde conduite (210 ; 310 ; 410 ; 510) à travers une conduite transversale inférieure (265 ; 365 ; 465 ; 565).

7. Porte-sonde modulaire selon l'une quelconque des revendications précédentes, **caractérisé en ce que** chacun desdits au moins deux modules porte-sonde (100 ; 200 ; 300 ; 400 ; 500) est un type de module porte-sonde (100 ; 200 ; 300 ; 400 ; 500) sélectionné dans un groupe comprenant au moins deux types de modules porte-sonde (100 ; 200 ; 300 ; 400 ; 500) qui sont différents et capables d'être couplés l'un à l'autre, éventuellement constitués d'une matière plastique transparente, encore plus éventuellement constitués de polyméthacrylate de méthyle.

8. Module porte-sonde (100 ; 200 ; 300 ; 400 ; 500) destiné à être utilisé dans le porte-sonde modulaire selon l'une quelconque des revendications 1 à 7, configuré pour loger au moins une sonde de détection, qui inclut une conduite (110 ; 210 ; 310 ; 410 ; 510) reliée à une entrée (120 ; 260 ; 360 ; 460 ; 560) et à une sortie (130 ; 230 ; 330 ; 430 ; 530), le module porte-sonde (100 ; 200 ; 300 ; 400 ; 500) étant configuré pour recevoir un écoulement d'un fluide de l'entrée (120 ; 260 ; 360 ; 460 ; 560) à la sortie (130 ; 230 ; 330 ; 430 ; 530) et ayant une première paroi latérale et une seconde paroi latérale, au moins l'une de l'entrée (120 ; 260 ; 360 ; 460 ; 560) et de la sortie (130 ; 230 ; 330 ; 430 ; 530) étant sur l'une entre les première et seconde parois latérales, le module porte-sonde (100 ; 200 ; 300 ; 400 ; 500) étant **caractérisé en ce qu'**il est doté, sur au moins l'une entre les première et seconde parois latérales, d'au moins un siège (650 ; 660 ; 670 ; 680) configuré pour recevoir un moyen mécanique de fixation (700 ; 750).

9. Module porte-sonde (100 ; 200 ; 300 ; 400 ; 500) selon la revendication 8, **caractérisé en ce que** ledit au moins un siège (650 ; 660 ; 670 ; 680) est configuré pour recevoir partiellement au moins une attache (700), ledit au moins un siège (650 ; 660 ; 670 ; 680) communiquant avec au moins un trou fileté (610 ; 630 ; 15) agencé sur une paroi avant ou arrière du module porte-sonde (100 ; 200 ; 300 ; 400 ; 500) et configuré pour recevoir au moins une vis sans tête (710).

10. Module porte-sonde (100 ; 200 ; 300 ; 400 ; 500) selon la revendication 8, **caractérisé en ce que** ledit au moins un siège (650 ; 660 ; 670 ; 680) est vissé et il est configuré pour recevoir au moins un goujon fileté (750).

11. Module porte-sonde (100 ; 200 ; 300 ; 400 ; 500) selon l'une quelconque des revendications 8 à 10, **caractérisé en ce qu'**il est doté en outre, sur au moins l'une entre les première et seconde parois latérales sur lesquelles l'entrée (120 ; 260 ; 360 ; 460 ; 560) ou la sortie (130 ; 230 ; 330 ; 430 ; 530) est présente, d'au moins une rainure de joint d'étanchéité (800) configurée pour recevoir au moins un joint d'étanchéité élastique (810) et agencée pour entourer l'entrée (120 ; 260 ; 360 ; 460 ; 560) ou la sortie (130 ; 230 ; 330 ; 430 ; 530) présente sur la paroi latérale dotée de ladite au moins une rainure de joint d'étanchéité (800), ladite au moins une rainure de joint d'étanchéité (800) ayant éventuellement une section transversale rectangulaire.

12. Module porte-sonde (100) selon l'une quelconque des revendications 8 à 11, **caractérisé en ce qu'**il est configuré pour loger un débitmètre (1100, 1110, 1120), comprenant éventuellement un siège creux arrière (150) configuré pour loger un capteur de proximité (1120), encore plus éventuellement un commutateur à lames ou un capteur à effet Hall, la conduite du module porte-sonde (100) étant une conduite verticale (110) configurée pour loger un flotteur (1100) interagissant avec un capteur de proximité (1120) lorsque ce dernier est logé dans le siège creux arrière (150), la sortie (130) du module porte-sonde (100) étant agencée sur l'une entre les première et seconde parois latérales, l'entrée du module porte-sonde (100) étant une ouverture inférieure (120) et la sortie (130) du module porte-sonde (100) étant agencée au niveau supérieur sur l'une entre les première et seconde parois latérales, la sortie (130) du module porte-sonde (100) étant reliée à la conduite verticale (110) à travers une conduite transversale supérieure (135), le module porte-sonde (100) ayant un siège creux latéral supérieur (140) dans lequel une vanne filetée de robinet de régulation (1200) a une pointe conique (1210), dont les génératrices sont inclinées selon un angle éventuellement compris entre 8° et 10° par rapport à un axe longitudinal de la vanne (1200), la conduite transversale supérieure (135) étant conique avec les génératrices inclinées selon un angle égal à l'angle d'inclinaison des génératrices de la pointe conique (1210) de la vanne (1200), la vanne (1200) ayant éventuellement un filetage avec un pas ne dépassant pas 1 mm.

13. Module porte-sonde (500) selon l'une quelconque des revendications 8 à 11, **caractérisé en ce que** la conduite du module porte-sonde (500) est une conduite verticale (510), le module porte-sonde (500) étant configuré pour loger un capteur ampérométrique (970) dans un siège creux inférieur (570) communiquant au niveau supérieur avec la conduite verticale (510), l'entrée (530) du module porte-sonde (500) comprenant une rainure (560) sur une paroi latérale d'entrée sélectionnée entre les première et seconde parois latérales, la rainure (560) communiquant avec la conduite verticale (510) à travers une conduite transversale inférieure (565), la sortie (530) du module porte-sonde (500) étant agencée au niveau supérieur sur une paroi latérale de sortie sélectionnée entre les première et seconde parois latérales différente de la paroi latérale d'entrée, le module porte-sonde (500) étant en outre doté d'une conduite transversale supérieure, éventuellement tubulaire, (580) qui met en communication une partie supérieure de la rainure (560) avec une partie supérieure de la conduite verticale (510).

14. Module porte-sonde (500) selon la revendication 13, **caractérisé en ce que** la conduite transversale inférieure (565) est reliée entre la rainure (560) et une ouverture inférieure (520) communicant à l'extérieur, le siège creux inférieur (570) étant configuré pour loger une pluralité de billes, le siège creux inférieur (570) communiquant avec l'ouverture inférieure (520) à travers une pluralité de conduites tubulaires internes (575) inclinées par rapport à un plan transversal traversant les première et seconde parois latérales, un angle d'inclinaison α de chaque conduite tubulaire interne (575) par rapport audit plan transversal étant éventuellement compris entre 20° et 30°, encore plus éventuellement égal à 25°, le siège creux inférieur (570) communiquant au niveau supérieur avec la conduite verticale (510) à travers une ou plusieurs conduites planes (515) orientées parallèlement à un axe longitudinal de la conduite verticale (510).

15. Module porte-sonde (500) selon l'une quelconque des revendications 8 à 14, **caractérisé en ce qu'**il est constitué d'une matière plastique transparente, éventuellement de polyméthacrylate de méthyle, le module étant éventuellement doté sur une paroi arrière d'au moins une paire de trous (850, 860), chaque trou (850, 860) étant configuré pour recevoir une vis pour fixer un ou plusieurs supports arrière.
